# EUROPEAN PATENT SPECIFICATION

(11) **EP 1 951 365 B1**
(45) Date of publication and mention of the grant of the patent: **04.10.2017**
(21) Application number: 06821561.5
(22) Date of filing: 16.11.2006
(51) Int. Cl.: A61N 1/18, A61N 1/04, A61N 1/36

(54) **GAIT MODULATION SYSTEM**
GANGMODULATIONSSYSTEM
SYSTEME DE MODULATION DE DEMARCHE

(30) Priority: 16.11.2005 US 736858 P; 27.04.2006 US 380430; 26.10.2006 US 552997
(43) Date of publication of application: 06.08.2008
(73) Proprietor: Bioness Neuromodulation Ltd, 43654 Raanana (IL)
(72) Inventor: DAR, Amit, 40692 Kfar Hess (IL); SHALEV, Yossef, 43506 Raanana (IL); BAR-OR, Jonathan, 37044 Pardes Hanna (IL); NATHAN, Roger, 46592 Herzlia B (IL)
(74) Representative: Gill Jennings & Every LLP
(86) International application number: PCT/IL2006/001326
(87) International publication number: WO 2007/057899

(56) References cited:
- US-A- 5 643 332
- US-A- 5 643 332
- US-A- 6 019 877
- US-A1- 2003 114 892
- US-A1- 2003 114 892
- US-A1- 2003 114 893
- US-A1- 2003 114 893
- US-A1- 2004 082 979
- US-A1- 2005 085 708
- US-A1- 2005 192 645
- US-A1- 2005 192 645

## Description

### FIELD AND BACKGROUND OF THE INVENTION

The present invention relates to gait modulation systems and methods therefor, and more particularly, to a functional electrical stimulation (FES) orthosis device and a sensor device for such gait modulation systems, and to method of using such devices.

It is known that pathologies of the neuromuscular system due to disease or trauma to the central nervous system, such as stroke, spinal cord injury, head injury, cerebral palsy and multiple sclerosis, can impede proper limb functioning of the hands or legs. Gait, the biomechanical description of walking, can suffer static and dynamic parameter variations due to neuromuscular impairments, which cause non-symmetrical walking, reduced walking speed and reduced walking stability.

Drop foot describes the gait attributable to weak or uncoordinated activation of the ankle dorsi-flexors due to disease or trauma to the central nervous system. Patients suffering from drop foot tend to drag the foot during the swing phase of walking and usually try to compensate for this dragging by hiking their hip or swinging it in a circular motion. These patients tend to have impaired stability, are prone to frequent falls, and their walking movements are unaesthetic and energy consuming.

However, limb muscles can generally be activated with FES. In FES, precisely timed bursts of short electrical pulses are applied to motor nerves, to generate muscle contraction, which can be applied to enhancing limb function.

Although more than four decades have elapsed since the first neuroprosthetic system was proposed, much room remains for improving the technological quality of such systems. This is reflected, inter alia, by the relatively small percentage of potential users who regularly don a neuroprosthetic device to compensate for limb and gait problems, and particularly, a drop foot problem. These systems suffer from many drawbacks that prevent them from being widely used by potential patients.

When problems with arm movement or gait result from stroke or brain injury, they are often accompanied by hand impairment on the same side of the body as the limb on which the FES orthosis is donned. Thus, the donning action must often be carried out using solely the contra-lateral, unaffected hand. Moreover, the posture of the plegic limb is often problematic, especially in cases where spasticity results in reduced voluntary movements and also in a limited passive range of motion of the limb joints. Consequently, objective biomechanical problems exist in donning the orthotic device and in locating the electrodes in exact position onto the limb. Prior art neuroprosthetic devices differ in that they fail to enable facile, quick and accurate donning of the device by an impaired patient using a single hand, and particularly, when even that hand is shaky or otherwise unstable.

Prior art external FES devices as e.g. US Patent Publications US2004/0082979 and US2003/0114892 typically utilize a stimulator unit that is physically separate from the FES orthosis, to create and control the electrical pulses being applied to motor nerves. The external stimulator unit, which is connected to the FES electrodes by several electrical wires, is located on the body of the user, and is typically attached to the belt of the user. These devices can be inconvenient for the user. Particularly cumbersome is the wiring, which is usually arranged to run along the leg under the clothing to connect the device components.

In addition, neuroprostheses require precise fitting for each individual patient, in an optimal fashion, by exactly identifying the stimulation points that cause contraction of the muscles and positioning and locking the electrodes thereto. Consequently, use of the known devices, which are configured and dedicated to the anatomy and needs of a particular individual, is limited to that individual only, and further requires considerable expertise to reconfigure the device for transfer to another patient.

U.S. Patent Nos. 5,643,332 and 5,814,093 to Stein disclose an assembly for functional electrical stimulation during movement, including a band, mountable on the leg, carrying all of the components of the assembly to provide a self-contained unit. The components include: electrodes for stimulating a leg nerve; a V-shaped plate for conforming with the leg's tibia to reproducibly position the band so that the electrodes are located over the nerve; a tilt sensor for measuring the angular position of the lower leg; a control circuit for processing the sensor signal information and emitting pulses through the electrodes to stimulate the leg in response to phases of body movement and a battery for supplying power to the tilt sensor, control circuit and stimulator. The disclosed band is made of stretchable, breathable material.

WalkAide™ is a commercially available FES device of Innovative Neurotronics, Inc., and is based on the technology disclosed by Stein. The orthosis component of the WalkAide™ is a narrow band made of a thermoplastic material that is molded to the limb anatomy of an individual user by heating and softening the thermoplastic material and subsequently fitting the contour to the contour of the underlying limb segment. Thus the shape and size of the device and the electrode positioning is custom-fitted to the leg of one user and individualized for the user. This procedure is carried out by a trained medical professional.

For a clinic or rehabilitation center serving a large number of users, it would be advantageous a device that can be transferred from patient to patient hygienically and with facility. Neuroprosthetic devices require a significant and time-consuming set-up procedure carried out by a trained medical professional to fit the device to the anatomy of the limb, position the electrodes accurately over the motor point, and adjust the stimulation parameters when transferring the device to another patient. Parts of the orthosis are in prolonged contact with the skin during a session of use, and existing devices have no provision for hygienically passing the orthosis from the leg of one patient on to another.

Prior art orthosis-based devices for the leg such as the WalkAide™ device operate with relatively small electrodes typically having a diameter of 25 - 30 mm and a surface area in contact with the skin of no more than about 5 - 7 cm² positioned relatively close together in the orthosis. This enables the orthosis to take the form of a relatively narrow band to accommodate the small electrodes and separation. However, activation of the leg muscles by electrical stimulation requires typically high stimulation currents. But the stimulation current passing through the electrode to the skin surface activates skin sensory receptors in addition to underlying excitable motor nerve and muscle tissue and the intensity of sensory activation will depend on the intensity of the current density passing through the skin surface. The level of muscle activation is often limited in the typical patient by his individual tolerance to activation of these skin pain sensors. For these patients, it would be advantageous to reduce the sensory discomfort by lowering the skin current density while maintaining levels of muscle activation. A further means to increase the contraction force of the activated muscles is to increase the distance separating the electrodes in the pair, particularly the distance along the length of the leg. This can result in the recruitment of more muscle fibers, resulting in increased activated rotation torque output from the ankle joint, without the necessity to use a high stimulation current intensity. This electrode geometry and arrangement for increasing muscle force output and reducing sensory discomfort is too large to fit within the prior art narrow band design of the orthosis. To date, no orthosis exists for accommodating surface electrodes of such size and configuration.

Furthermore, a means for accurately positioning the electrodes along the length of the leg becomes essential where the electrodes are significantly separated in this longitudinal direction, and accurate longitudinal positioning of the orthosis becomes mandatory to avoid activating unwanted muscles. Accommodating the large stimulation electrodes with a larger distance separating them particularly in the longitudinal direction requires housing these electrodes in an orthosis that is significantly wider, extending both proximally and distally along the length of the leg. The wide orthosis design introduces new problems concerned with fitting and self-placement. Moreover, the larger dimensions of the orthosis appreciably compromise the ability of the orthosis to fit the contour of the limb segment, especially during limb extensions, flexions and gait.

Some commercially available FES devices utilize a sensor, for disposing underneath the foot of the user, to trigger the electrical pulses being applied to the motor nerves. The sensor senses foot rise or foot strike and accordingly triggers the stimulation pulses administered to the motor nerves. The sensor device is physically distinct from the orthosis.

U.S. Patent 6,507,757 to Swain, et al., discloses one typical foot sensor device of the prior art, in which a foot pressure switch, or sensor, is permanently disposed in the shoe of the affected leg. An electrical circuit is interrupted during the stance phase, when a significant weight is placed on the heel, and reconnects when the heel is lifted during the swing phase. Wires disposed under the clothing connect the sensor with an external stimulator unit that can be attached to the belt or kept in a pocket of the user. The stimulator unit is connected to the electrodes by additional electrical wires.

In other FES orthotic devices, the cumbersome wires are obviated by using a radio frequency (RF) system in which the foot sensor device and other components of the FES orthotic device communicate in a wireless fashion. However, the use of such an RF system necessitates integrating an RF transmitting unit, or head, within the foot sensor device. The transmitting unit can be bulky and sensitive to humidity and mechanical stress. Consequently, such transmitting units are typically mounted on and attached to the calf of the patient.

U.S. Design Patent D494,273 to Haugland, et al., assigned to Neurodan A/S, describes a pressure sensor switch device for placing underneath the foot of the patient. The communication head is held at a predetermined distance from the sensor device by a wide, semi-rigid spine. The device disclosed by Haugland, et al., can be used as a component of the ActiGait® system manufactured by Neurodan A/S. In the ActiGait® system, the pressure sensor device is inserted into a small pouch within a sock, such that upon donning of the sock, the pressure switch is disposed underneath the foot. A substantially non-elastic band is tightened around the calf to secure the RF unit in place against the calf of the impaired leg.

This approach is disadvantageous in that the patient, who is often hemi-plegic or may suffer from other disorders, is required to add the donning of an additional item - the sock - to his routine. This unintuitive action is particularly problematic for patients who need or prefer to don the FES orthotic device in an unassisted fashion.

A further disadvantage of this design is that the semi-rigid spine may rub against the foot and heel. Also, the electronic head that houses the RF transceiving unit may rub the ankle or lower calf of the user during the course of gait. Moreover, because the pressure switch and RF transceiving unit are mechanically connected by a wide, at least semi-rigid neck, pressures exerted on the electronic head (e.g., when the head gets caught on, or bumped by, an object during the course of gait), are mechanically translated into forces on the foot sensor. These forces may impede the efficacy and sensitivity of the foot sensor. A further disadvantage lies in the limited ability to adjust the electronic head of the pressure switch to different shoe heights: the electronic head protrudes excessively from low-rimmed shoes, and cannot be fitted to shoes or boots in which the rim is at or above the height of the electronic head.

There is therefore a recognized need for, and it would be highly advantageous to have, a sensor device for neuroprosthetic gait enhancement that overcomes the various deficiencies of the prior art devices. It would be of particular advantage to have such a sensor device that is essentially effortless to don, avoids the discomfort associated with prior art sensor devices, and is secured so as to operate in a safe and robust fashion.

It would also be highly advantageous to have an improved FES orthosis for a neuroprosthetic system and method that overcome the manifest deficiencies of the prior art. It would be advantageous to have an FES leg orthosis that can easily and accurately be donned on the limb by patients also suffering from an impaired hand. It would also be of particular advantage to have an FES leg orthosis in which an even pressure of the electrode surface is maintained during limb extensions, flexions and gait. It would be of further advantage to enable greater ankle torque generation with lessening of skin sensory discomfort at the electrode site by increasing the size and longitudinal separation of the electrodes. It would be of yet further advantage for the FES orthosis to be substantially universally adaptable to the different anthropometric variables of limbs and to electrode positioning needs of a wide variety of users. Finally, it would be advantageous to have an FES orthosis designed and configured such that the on-board stimulation unit does not significantly protrude outside the profile of the orthosis and does not impede donning and wearing clothing such as trousers over the orthosis. This is of major significance to the stroke patient, who is generally is challenged by donning trousers on to his plegic leg using a single hand, and a protruding device attached to his leg may disable his ability to dress himself independently.

### SUMMARY OF THE INVENTION

While the invention is defined in claim 1, in the following description there is provided an orthosis for providing electrical stimulation to a limb segment of a user, including: (a) an at least semi-rigid, self-retaining C-shaped frame, the frame configured to substantially envelop the limb segment, the frame including a first flexible and elongated circumferentially retaining element and at least a first and a second opposing flexible and elongated circumferentially retaining elements disposed on the circumferentially opposite side of the frame, the first retaining element and the first opposing retaining element forming a pair of opposing retaining elements, and (b) at least one surface electrical stimulation electrode for contacting at least one stimulation point on a surface of the limb segment, associated with, and supported by, the frame, the at least one surface electrode for electrically associating, via the frame, with a stimulator unit, so as to provide electrical stimulation, wherein the opposing retaining elements are configured to be radially spring-loaded towards a center of the frame, such that in donning the orthosis around the limb segment, the limb segment applies a counter-pressure from within the frame, against the opposing retaining elements, such that the orthosis is firmly and fixedly self-retained in a pre-determined position on the surface of the limb segment.

According to further features in the described preferred embodiments, all of the flexible and elongated retaining elements are configured to conform to a contour of the surface in a substantially independent fashion, so as to maintain intimate contact with the contour.

According to still further features in the described preferred embodiments, the opposing retaining elements include at least three flexible and elongated circumferentially retaining elements.

According to still further features in the described preferred embodiments, the orthosis further includes: (c) a locking mechanism, associated with the frame, for locking the at least one surface electrical stimulation electrode at the pre-determined position on the surface of the limb segment.

According to still further features in the described preferred embodiments, the opposing retaining elements are designed and configured to independently respond, mechanically, to changes in the contour, so as to retain the at least one surface electrical stimulation electrode fixed against the pre-determined position on the limb segment and so as to maintain an even pressure against the pre-determined position on the surface of the limb segment.

According to still further features in the described preferred embodiments, the semi-rigid frame includes a housing for receiving the stimulator unit.

According to still further features in the described preferred embodiments, the housing is dimensioned to envelop and hold the stimulator unit intimately and flatly against the orthosis.

According to still further features in the described preferred embodiments, the orthosis further includes: (c) the stimulator unit, wherein the stimulator unit is designed and configured to: (i) communicate with a sensor for sensing a physical parameter relating to the limb segment, and (ii) based on a signal relating to the sensor, deliver a stimulation signal to the surface electrode.

According to still further features in the described preferred embodiments, the stimulator unit includes a radio frequency transceiver for communicating with at least one of a stimulator control unit and the sensor.

According to still further features in the described preferred embodiments, the locking mechanism includes at least one elastic strap designed to extend circumferentially around and bridge between the opposite sides of the frame and to be reversibly fastened to a fastening element associated with the frame.

According to still further features in the described preferred embodiments, the orthosis is a lower-leg orthosis, an upper contour of the orthosis is a locating surface, the locating surface configured to conform to an inferior border of a patella of the user.

According to still further features in the described preferred embodiments, the locating surface has a three-dimensional cup shape for abutting an inferior border of a patella of the user during donning of the orthosis.

According to still further features in the described preferred embodiments, the orthosis is adapted for single-handed donning by the user.

According to still further features in the described preferred embodiments, the fastening element includes a protuberance associated with the frame.

According to still further features in the described preferred embodiments, the protuberance includes a housing for receiving the stimulator unit.

According to still further features in the described preferred embodiments, the at least one elastic strap terminates in a looped handle, the looped handle adapted to be reversibly fastened to the fastening element.

According to still further features in the described preferred embodiments, the orthosis further includes: (c) the neuroprosthetic stimulator unit, and (d) a housing for receiving the stimulator unit, the housing disposed on a flexible leaf on the frame, such that the housing and the leaf are free to move as a unit, independently of the retaining elements, so as enable the retaining elements to mechanically respond, substantially independently, to changes in a contour of the leg segment, even when pressure is exerted on an exterior face of the stimulator unit.

According to still further features in the described preferred embodiments, circumferentially disposed on the frame is at least one spring-loaded strip, the strip being radially spring-loaded towards a center of the frame, such that in donning the orthosis around the limb segment, the spring-loaded strip applies a pressure against the limb segment, such that the orthosis is self-retained in position on the limb segment while allowing for small adjustments to be made in positioning of the orthosis on the limb segment.

According to still further features in the described preferred embodiments, the second flexible and elongated circumferentially retaining element has a width, W, wherein W is within a range of about 2 - 4.5 cm.

According to still further features in the described preferred embodiments, the second opposing retaining element has a width, W, and the orthosis has a height, H, and wherein W is within a range of 8 - 60% of H.

According to still further features in the described preferred embodiments, each of the at least one surface electrical stimulation electrode has a surface area for contacting the surface of the limb segment, and wherein for each electrode, the surface area is at least 9 cm2.

According to still further features in the described preferred embodiments, the surface area is at least 12 cm2.

According to still further features in the described preferred embodiments, the at least one elastic strap terminates in a looped handle, the looped handle designed to be reversibly fastened to the fastening element, and wherein the looped handle, when fastened to the fastening element, completes a smooth and substantially non-protruding profile that includes the stimulator unit, the housing, and the looped handle, all of which together blend into a profile of a leg of the user.

According to still further features in the described preferred embodiments, the stimulator unit is a neuroprosthetic stimulator unit.

According to another aspect there is provided an orthosis for providing electrical stimulation to a limb segment of a user, the orthosis including: (a) an at least semi-rigid frame being configured to substantially envelop the limb segment; (b) a soft inner facing for at least partially covering an inner face of the frame and for providing a comfortable interface between the frame and the limb segment; (c) a first mechanical fitting associated with the inner face of the frame, and (d) at least one surface electrical stimulation electrode assembly associated with, and supported by the frame, the assembly having a stimulation electrode having a first surface for contacting at least one stimulation point on the limb segment, and an attachment mechanism for fixing a position of the electrode with respect to the frame, the at least one surface electrode for electrically associating, by way of the frame, with a stimulator unit for providing a stimulation signal to the surface electrode, and wherein the stimulation electrode assembly has a second mechanical fitting, complementary to the first mechanical fitting, for reversibly attaching the stimulation electrode assembly to, and reversibly detaching the stimulation electrode assembly from, the first mechanical fitting.

According to further features in the described preferred embodiments, the first and second mechanical fittings are connectors selected from the group consisting of snaps and hook and loop fasteners.

According to still further features in the described preferred embodiments, the attachment mechanism includes an electrode base having a surface for receiving and engaging a second surface of the stimulation electrode, the second surface being opposite the first surface.

According to still further features in the described preferred embodiments, the electrode base has a rim for physically defining, for the stimulation electrode, a substantially singular position therein.

According to still further features in the described preferred embodiments, the second surface of the stimulation electrode includes a hydrogel-containing surface, and wherein the surface of the electrode base includes at least one patch of hook fasteners for securing the hydrogel-containing surface to the electrode base.

According to still further features in the described preferred embodiments, the soft inner facing is a reversibly attachable and detachable panel.

According to still further features in the described preferred embodiments, the orthosis further includes: (e) a soft panel for covering at least a portion of the soft inner facing, the soft panel having a third complementary connector for associating with a fourth complementary connector associated with the frame, so as to reversibly secure the soft panel to the frame.

According to still further features in the described preferred embodiments, the attachment mechanism includes an electrode base having a surface for attaching to a second surface of the stimulation electrode, the second surface being opposite the first surface.

According to still further features in the described preferred embodiments, the electrode base has a cover, attached to the base, the cover for protecting a perimeter of the stimulation electrode.

According to still further features in the described preferred embodiments, the cover is adapted so as to leave exposed a majority of the first surface.

According to still further features in the described preferred embodiments, the perimeter includes hydrogel.

According to yet another aspect there is provided an orthosis for providing electrical stimulation to a limb segment of a user, the orthosis including: (a) an at least semi-rigid frame configured to substantially envelop the limb segment, the frame having at least one first complementary mechanical fastener associated therewith; (b) at least one surface electrical stimulation electrode assembly associated with, and supported by the frame, the assembly having a surface stimulation electrode for contacting at least one stimulation point on the limb segment, the at least one surface electrode assembly having an electrode base for electrically associating, via the frame, with a stimulator unit for providing a stimulation signal to the surface electrode, the electrode base having a top face for receiving the stimulation electrode, the electrode base having a bottom face having at least one second complementary mechanical fastener, the second fastener being complementary to the first fastener, the first and second fasteners designed and configured for reversible attachment and reversible detachment, at a plurality of locations on the frame, thereby enabling the electrical stimulation electrode assembly to be adjustably and reversibly positioned on the frame.

According to further features in the described preferred embodiments, the electrode base is associated with a conductive element for electrically connecting the base to the stimulator unit.

According to still further features in the described preferred embodiments, the conductive element is a first conductive complementary mechanical fastener, and wherein associated with the frame is a second conductive complementary mechanical fastener, the first conductive fastener being complementary to the second conductive fastener.

According to still further features in the described preferred embodiments, the electrode base is loosely associated with the first conductive fastener by means of a flexible wire, thereby enabling the electrical stimulation electrode assembly to be adjustably and reversibly positioned on the frame in a plurality of positions, according to individual needs of the user.

According to still further features in the described preferred embodiments, the electrode base is associated with the first conductive fastener by means of a flexible wire, thereby substantially decoupling an electrical connection of the electrode assembly to the stimulator unit from a mechanical connection of the electrode assembly to the stimulator unit, so as to enable the electrode assembly to be adjustably and reversibly positioned on the frame in a plurality of positions, according to individual needs of the user.

According to still further features in the described preferred embodiments, the first and second fasteners include hook and loop fasteners.

According to yet another aspect there is provided an orthosis for providing electrical stimulation to a limb segment of a user, the orthosis including: (a) a frame configured to substantially envelop a limb segment of a user, the frame being made of an at least semi-rigid material and associated with a first fastener at an inner face of the frame; (b) at least one electrical stimulation electrode assembly associated with, and supported by the frame, the stimulation electrode assembly for electrical association, via the frame, with a stimulator unit; (c) a soft, reversibly attachable and detachable layer having a second fastener complementary to the first fastener for reversible attachment and detachment of the layer from an inner face of the frame, the at least one electrical stimulation electrode assembly being associated with, and located on, an inner face of the layer such that, when the orthosis is donned on the limb segment, an electrical stimulation electrode of the electrode assembly is positioned so as to contact at least one stimulation point of a muscle of the user, the soft, reversibly attachable and detachable layer for providing the user with a comfortable interface between the frame and the limb segment during donning of the orthosis.

According to further features in the described preferred embodiments, the first fastener and second fastener include a hook and loop arrangement.

According to still further features in the described preferred embodiments, the layer further includes a snap connector for attaching the layer to the frame, the snap connector being complementary to a connector associated with the frame so as to provide a secure mechanical attachment to the frame, thereby ensuring exact and repeatable positioning of the layer, with respect to the frame.

According to still further features in the described preferred embodiments, the layer further includes a hollow snap connector, the hollow connector being complementary to a recess associated with the frame so as to provide a tight mechanical attachment to the frame, thereby ensuring exact and repeatable positioning of the layer, with respect to the frame.

According to still further features in the described preferred embodiments, the layer further includes a hole for enabling the electrical association.

According to still further features in the described preferred embodiments, the electrical stimulation electrode assembly includes a connector designed to penetrate the hole and to connect to a complementary connector associated with the frame.

According to still further features in the described preferred embodiments, the reversibly attachable and detachable layer is fully flexible.

According to yet another aspect there is provided an orthosis for providing electrical stimulation to a limb segment of a user, the orthosis including: (a) a frame adapted to substantially envelop a limb segment of a user, the frame being made of an at least semi-rigid material and associated with a first fastener at an inner face of the frame; (b) at least one electrical stimulation electrode assembly associated with, and supported by the frame, the stimulation electrode assembly for electrically associating, via the frame, with a stimulator unit, the assembly including an electrical stimulation electrode and a stimulation electrode base; (c) a reversibly attachable and detachable layer having a second fastener complementary to the first fastener for reversible, yet secure mechanical attachment and detachment of the layer to and from an inner face of the frame, the at least one stimulation electrode base being attached to, and located on, an inner face of the layer, such that, when the orthosis is donned on the limb segment, the electrical stimulation electrode is positioned to contact at least one stimulation point of a muscle of the user, and wherein the electrode assembly and the layer are designed and configured so as to enable positioning and securing of the electrode assembly on the layer in a plurality of positions, according to individual needs of the user.

According to further features in the described preferred embodiments, the electrode assembly and the layer are further designed and configured such that the positioning is adjustable and reversible.

According to still further features in the described preferred embodiments, the electrode base has at least a first connector, and the layer has at least a second connector, the second connector being complementary to the first connector, so as to achieve the positioning and securing.

According to still further features in the described preferred embodiments, the electrode assembly and the layer are further designed and configured such that the positioning is adjustable and reversible.

According to still further features in the described preferred embodiments, the electrode base has a loose association with the first connector, so as to enable the positioning and securing of the electrode assembly on the layer in the plurality of positions.

According to still further features in the described preferred embodiments, the loose association is achieved by a flexible wire.

According to still further features in the described preferred embodiments, the reversibly attachable and detachable layer is fully flexible.

According to yet another aspect there is provided a method of providing electrical stimulation to a limb segment of a user, the method including the steps of: (a) providing a electrical stimulation device including an electrical stimulation orthosis, the orthosis including: (i) a frame being configured to substantially envelop a limb segment of a user, the frame being made of an at least semi-rigid material and associated with a first fastener at an inner face of the frame; (ii) at least one electrical stimulation electrode associated with, and supported by, the frame, the stimulation electrode being electrically associating, via the frame, with a stimulator unit; (iii) a soft, reversibly attachable and detachable layer having a second fastener complementary to the first fastener for reversibly attaching and detaching the layer from an inner face of the frame, the at least one electrical stimulation electrode being associated with, and located on, an inner face of the layer so as to contact at least one stimulation point of a muscle of the user, and (b) donning the electrical stimulation device on the limb segment.

According to still further features in the described preferred embodiments, the method further includes the step of: (c) reversibly repositioning the electrical stimulation electrode on the inner face of the layer.

According to still further features in the described preferred embodiments, the method further includes the step of: (c) reversibly detaching the layer from the inner face of the frame.

According to yet another aspect of the present invention there is provided an electrode assembly including a surface electrode having: a hydrogel layer having a first face and a second face, the second face substantially opposite the first face, and at least one underlying surface layer, associated with the first face of the hydrogel layer, a supporting surface layer, disposed along the second face, the supporting surface layer attached to the underlying surface layer so as to provide mechanical reinforcement of the hydrogel layer.

According to further features in the described preferred embodiments, the supporting surface layer is adapted to reinforce a perimeter of the hydrogel layer.

According to still further features in the described preferred embodiments, the at least one underlying surface layer includes at least a first layer disposed directly against the hydrogel layer, and a second layer disposed against the first layer, and wherein the second layer is attached to the supporting surface layer.

According to still further features in the described preferred embodiments, the supporting surface layer includes polypropylene.

According to still further features in the described preferred embodiments, the second layer includes polypropylene.

According to still further features in the described preferred embodiments, the second layer includes hook or loop fasteners.

According to still further features in the described preferred embodiments, the supporting surface layer and the underlying surface layer are attached through the hydrogel.

According to still further features in the described preferred embodiments, the supporting surface layer and the underlying surface layer are attached by a glue.

According to still further features in the described preferred embodiments, the supporting surface layer and the underlying surface layer are attached by a weld.

According to still further features in the described preferred embodiments, the supporting surface layer and the underlying surface layer are attached by an ultrasonic weld.

According to still further features in the described preferred embodiments, the second layer includes a non-woven material.

According to still further features in the described preferred embodiments, the supporting surface layer includes a non-woven material.

According to still further features in the described preferred embodiments, the supporting surface layer and the underlying surface layer are attached around a perimeter of the hydrogel.

According to still further features in the described preferred embodiments, the supporting surface layer is substantially ring-shaped.

According to yet another aspect there is provided a foot sensor device for gait enhancement, including: (a) a sensor unit having an external casing, the sensor unit for disposing within a shoe of a user, the sensor unit for sensing a parameter associated with a gait event; (b) an electronic communication unit, electrically associated with the sensor unit, for receiving a signal pertaining to the parameter, the electronic unit having: (i) a microcontroller; (ii) a transmitting unit for transmitting, in a wireless fashion, gait information based on the signal, to a unit of the orthosis external to the foot sensor device, and (iii) a housing for housing at least one of the microcontroller and the transmitting unit, and (c) a fastening unit, attached to the housing, the fastening unit adapted to fasten on to the shoe, so as to secure the electronic communication unit in a substantially fixed position during gait of the user.

According to still further features in the described preferred embodiments, the fastening unit includes a clamp unit.

According to still further features in the described preferred embodiments, the fastening unit is adapted to fasten on to a rim of the shoe.

According to still further features in the described preferred embodiments, the clamp unit has at least two arms designed and configured to fasten around a rim of the shoe.

According to still further features in the described preferred embodiments, the clamp unit is at least semi-rigidly associated with the housing.

According to still further features in the described preferred embodiments, the clamp unit is rigidly associated with the housing.

According to still further features in the described preferred embodiments, the housing of the electronic unit and the casing of the sensor unit are substantially mechanically independent.

According to still further features in the described preferred embodiments, the clamp unit further includes a locking lever adapted for locking the arms in position around the rim of the shoe.

According to still further features in the described preferred embodiments, a facing of the housing is concave, so as to fit a natural curvature of a leg of the user.

According to still further features in the described preferred embodiments, the housing and the clamp unit each have a face for disposing towards a leg of the user when the user wears the shoe, the device being designed and configured such that the housing and the clamp unit are attached to form an angle within a range of about 150° to about 175° between the faces.

According to still further features in the described preferred embodiments, the angle is in a range of about 155° to about 175°.

According to still further features in the described preferred embodiments, the angle is in a range of about 160° to about 175°.

According to still further features in the described preferred embodiments, the angle is in a range of about 160° to about 170°.

According to still further features in the described preferred embodiments, the transmitting unit is a transceiving unit.

According to still further features in the described preferred embodiments, the foot sensor device further includes: (d) the shoe.

According to still further features in the described preferred embodiments, the sensor unit is disposed under an inner sole of the shoe.

According to still further features in the described preferred embodiments, the fastening unit includes a clamp unit adapted to enable a hemiplegic patient to affix the clamp unit to the shoe, using a single hand.

According to yet another aspect there is provided a method of enhancing gait, including the steps of: (a) providing a device including: (i) a sensor unit having an external casing, the sensor unit for disposing within a shoe of a user, the sensor unit for sensing a parameter associated with a gait event; (ii) an electronic communication unit, electrically associated with the sensor unit, for receiving a signal pertaining to the parameter, the electronic unit having: (A) a microcontroller; (B) a transmitting unit for transmitting, in a wireless fashion, gait information based on the signal, to a unit of the orthosis external to the foot sensor device, and (C) a housing for housing at least one of the microcontroller and the transmitting unit, and (iii) a fastening unit, attached to the housing, the fastening unit adapted to fasten on the shoe, so as to secure the electronic communication unit in a substantially fixed position during gait of the user, and (b) donning the device by affixing the fastening unit to the shoe.

According to still further features in the described preferred embodiments, the affixing of the fastening unit includes affixing of a clamp unit.

According to still further features in the described preferred embodiments, the fastening unit is affixed to a rim of the shoe.

According to still further features in the described preferred embodiments, the clamp unit has at least two arms that fasten around a rim of the shoe.

According to still further features in the described preferred embodiments, the housing of the electronic unit and the casing of the sensor unit are substantially mechanically independent.

According to still further features in the described preferred embodiments, the method of claim 95, further including, prior to step (b), the step of: (c) disposing the sensor unit under an inner sole of the shoe.

According to still further features in the described preferred embodiments, the affixing of the fastening unit is effected using a single hand.

According to still further features in the described preferred embodiments, the affixing of the fastening unit is effected using a single hand.

According to still further features in the described preferred embodiments, the affixing of the fastening unit is effected by a hemiplegic patient using a single hand.

According to still further features in the described preferred embodiments, the method further includes the step of: (c) locking the arms in position against the rim of the shoe.

According to yet another aspect there is provided a method of configuring operating parameters of an electrical stimulation orthosis system, including the steps of: (a) providing a personal digital assistant including: (i) a central processing unit having a memory; (ii) an input device; (iii) an operating system interfacing between the input device and the central processing unit, and (iv) configuration software, for configuration of the orthosis system, having specific interface for producing the configuration commands; (b) providing a control unit for controlling the electrical stimulation orthosis, and (c) providing a housing having at least one receptacle for receiving the personal digital assistant and the control unit, the housing adapted to electrically connect between the personal digital assistant and the control unit, (d) inserting the personal digital assistant and the control unit into the housing, so as to electrically connect between the personal digital assistant and the control unit; (e) attaching the electrical stimulation orthosis to a limb of a user, (f) activating the electrical stimulation orthosis so as to effect stimulation of the limb, and, while the personal digital assistant and the control unit are electrically connected in the housing, (g) monitoring at least one operating parameter associated with the electrical stimulation orthosis system, using the personal digital assistant, and (h) providing configuration commands, by the personal digital assistant, via the control unit, so as to configure the electrical stimulation orthosis, such that the control unit transmits the configuration commands and monitors operating parameters from the electrical stimulation orthosis system, during the stimulation of the limb.

According to still further features in the described preferred embodiments, the at least one operating parameter includes a stimulation profile.

According to still further features in the described preferred embodiments, the at least one operating parameter includes stimulation synchronization.

According to still further features in the described preferred embodiments, the at least one operating parameter includes user history of the user.

According to still further features in the described preferred embodiments, the user history includes at least one previous configuration associated with the user.

According to still further features in the described preferred embodiments, the user history includes an amount of use of the system by the user.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. Throughout the drawings, like-referenced characters are used to designate like elements.

In the drawings:
Figure 1 is a perspective view of the inventive FES orthosis for gait modulation;
Figure 2 is a perspective front view of a central, semi-rigid frame of the inventive FES orthosis of Figure 1;
Figure 3 is a perspective back view of the frame of Figure 2, showing also an attached stimulator unit and housing;
Figure 3A is a perspective side view of the frame of the FES orthosis of Figure 2, with the stimulator unit and housing removed to reveal a flexible leaf, integral to the frame, for supporting the stimulator unit housing;
Figure 4 is a perspective view showing a tightening mechanism of the inventive FES orthosis;
Figure 5 illustrates a side view of the donning of the device of Figure 1 on an impaired leg;
Figure 6 illustrates a front view of the donning of the device of Figure 1 on the impaired leg;
Figure 7 is a perspective view showing the attachment of a neuroprosthetic stimulator unit to the FES orthosis of Figure 1;
Figure 8 illustrates the association of the neuroprosthetic stimulator unit with a cradle of the FES orthosis of Figure 1;
Figure 8A is a perspective view showing a neuroprosthetic stimulator unit and a complementary receptacle or cradle for disposing on the FES orthosis, according to a preferred embodiment of the present invention;
Figure 9 is a perspective view of an electrode assembly of the inventive FES orthosis of Figure 1;
Figure 9A is a perspective view of another embodiment of the inventive electrode assembly;
Figure 9B is a schematic side view of a portion of the inventive FES orthosis, showing electrical and mechanical connections between the layers of the orthosis;
Figure 9C is a schematic perspective view of an electrode assembly having an electrode base and a ring-shaped cover, according to another embodiment of the present invention;
Figure 9D is a schematic top view of the inventive electrode assembly of Figure 9C;
Figure 10 is a perspective view of two electrode assemblies of Figure 9 attached to an internal soft layer of the device of Figure 1;
Figure 11 is a block diagram showing the main features of the electrical stimulation unit;
Figure 12A is a front view of a detachable layer of the FES orthosis, according to one aspect of the present invention;
Figure 12B illustrates another embodiment of the inventive detachable layer, having hollow snap connectors for securing the detachable layer to the internal layer of the orthosis, and
Figure 12C illustrates another embodiment of the inventive detachable layer, in which the layer covers only a portion of the surface of the internal layer of the orthosis;
Figure 12D is a schematic cross-sectional side view of a slice of a portion of the inventive FES orthosis, showing some of the electrical and mechanical connections between the detachable lining and other components of the orthosis;
Figure 12E is a schematic cross-sectional side view of a slice of a portion of the inventive FES orthosis, showing some of the electrical and mechanical connections between the detachable lining and other components of the orthosis, wherein the detachable lining is directly attached to the orthosis frame;
Figure 12F illustrates another embodiment of the inventive detachable layer, in which the layer is stiffened by at least one supporting region;
Figure 13A is a schematic perspective view of the electrode assembly and detachable layer, according to another embodiment of the present invention;
Figure 13B is a detailed magnification of Figure 13A;
Figure 13C is a detailed, magnified, cross-sectional view of a snap connector of the electrode assembly mechanically connected to a complementary connector on the detachable layer;
Figure 14 is a schematic exploded view of the electrode according to another aspect of the present invention;
Figure 15a is a perspective view of the inventive foot sensor device;
Figure 15b is a schematic, three-dimensional view of the inventive foot sensor device, the sensor unit being disposed underneath the shoe insole, and the communication unit being mounted on the shoe rim;
Figure 15c is a partial cross-sectional view of a shoe floor, a sensor casing, and a hook and loop system for affixing the casing to the floor;
Figure 16 is a schematic electronic diagram of the inventive foot sensor device;
Figure 17 is a perspective side view of the communication unit and the clamp associated therewith, the clamp disposed in an open position;
Figure 18 is another perspective side view of the communication unit and the clamp associated therewith, the clamp disposed in a closed position;
Figure 19a is a schematic, three-dimensional view of the inventive foot sensor device mounted on the shoe rim, with the communication head hugging the inner calf of the user;
Figure 19b is a schematic, three-dimensional top view of Figure 19a, and
Figure 20 is a schematic perspective drawing of an inventive configuration cradle for on-line configuration of the system by the clinician.

### DESCRIPTION OF THE PREFERRED EMBODIMENTS

One aspect of the present invention is an improved electrical stimulation orthosis and method, and more particularly, a functional electrical stimulation (FES) orthosis for users suffering from gait problems such as drop foot. The orthosis can easily be donned on the leg, even by patients suffering from an impaired hand.

Before explaining at least one embodiment of the invention in detail, it is to be understood that the invention is not limited in its application to the details of construction and the arrangement of the components set forth in the following description or illustrated in the drawing. The invention is capable of other embodiments or of being practiced or carried out in various ways. Also, it is to be understood that the phraseology and terminology employed herein is for the purpose of description and should not be regarded as limiting.

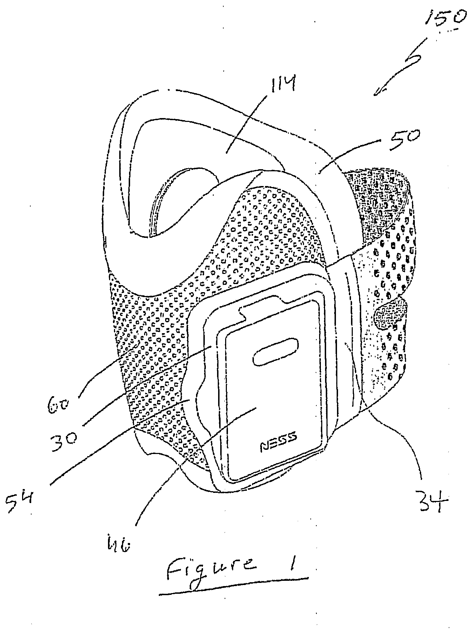

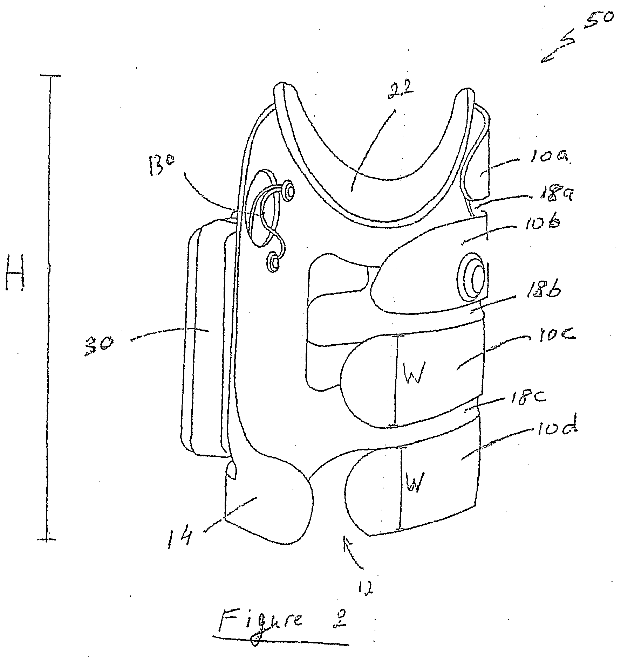

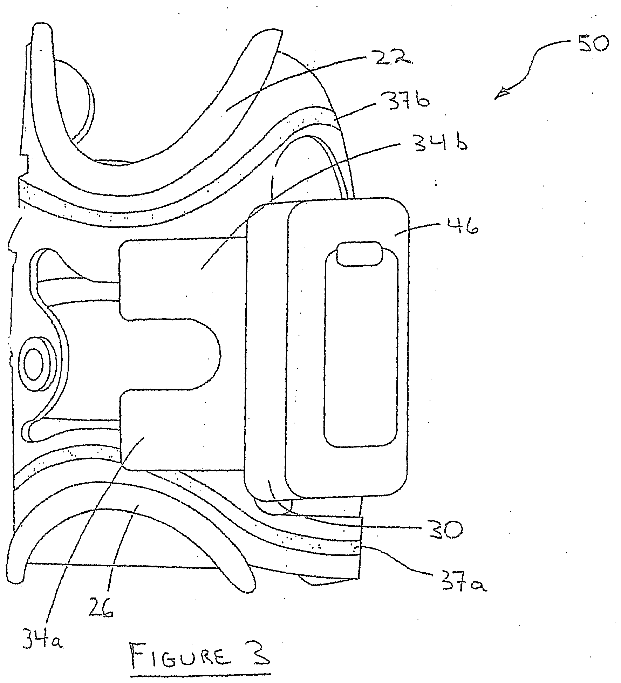

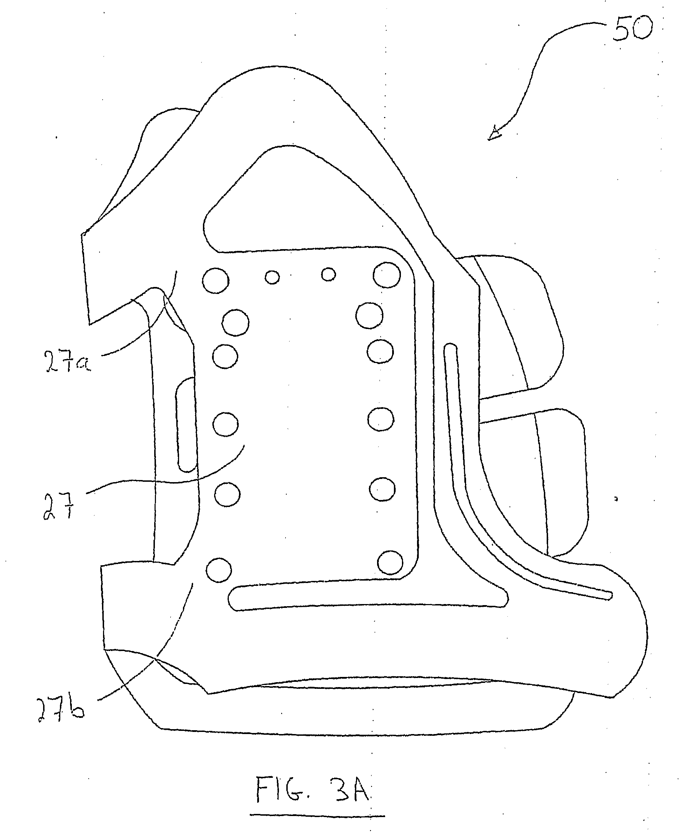

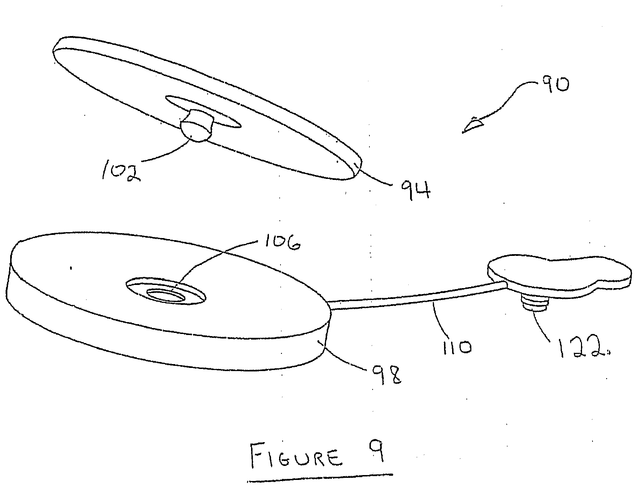

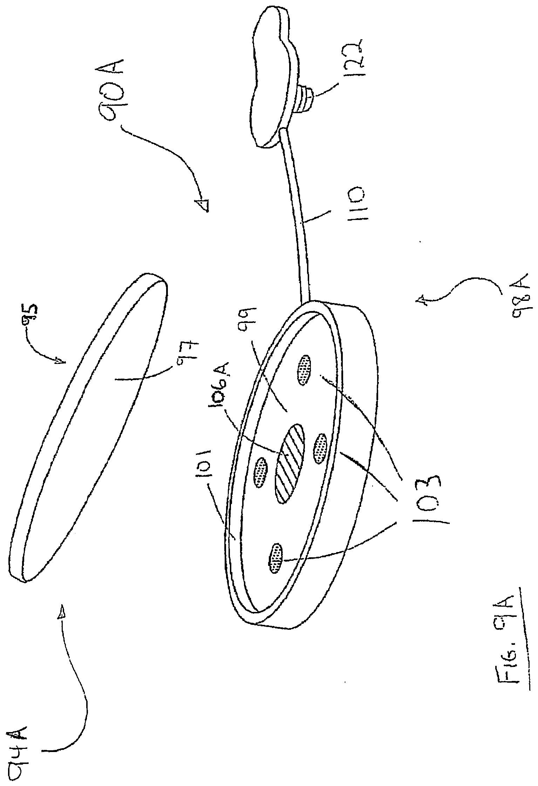

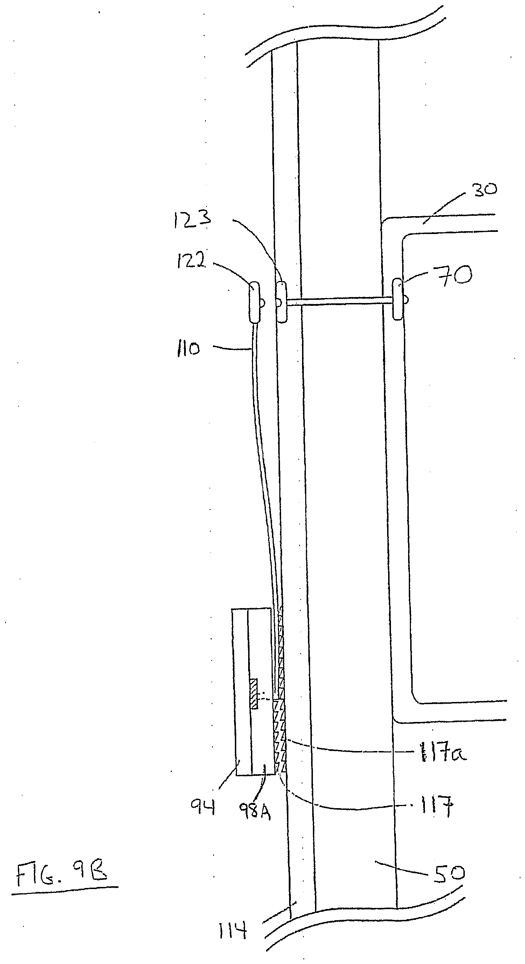

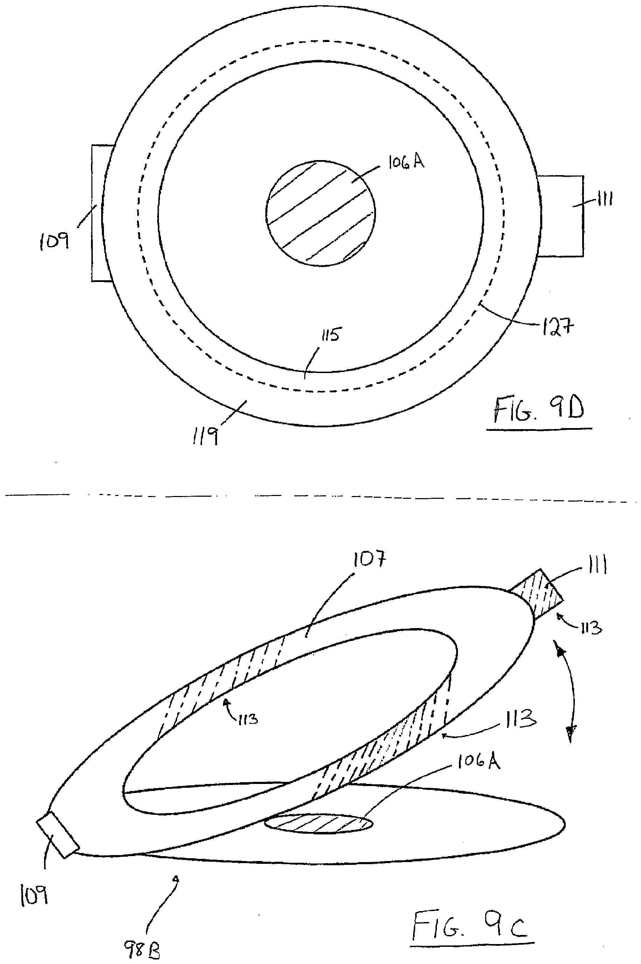

Referring now to the drawings, Figure 1 is a perspective view of the inventive FES gait modulation orthosis **150;** Figure 2 and Figure 3 are perspective front and back views, respectively, of the central, semi-rigid frame **50** of the FES orthosis. It can be seen from these drawings that FES orthosis **150** includes three layers: central frame **50,** which is at least semi-rigid, an internal soft layer **114** covering the inner facing of frame **50,** and an external soft layer **60** covering the outer facing of frame **50.** Additionally, orthosis **150** includes a neuroprosthetic stimulation unit **46,** as well as stimulation electrode assemblies (shown in detail in Figure 9 and described hereinbelow).

As used herein in the specification and in the claims section that follows, the term "limb segment" refers to a limb segment including a portion of the upper or lower arm, or the upper or lower leg.

As used herein in the specification and in the claims section that follows, the term "envelop", "enveloping", and the like, with regard to a limb segment and an article therefor, refers to an article that substantially surrounds and covers at least one half the circumference of a limb segment.

As used herein in the specification and in the claims section that follows, the term "reversible", "reversibly", and the like, with respect to attachment and/or detachment of an element or assembly, refers to a non-destructive, repeatable attachment and/or detachment. The term "reversible attachment", "reversibly attached", and the like, with respect to a soft layer associated with the frame of the orthosis, further refers to a reproducible positioning of the soft layer, with respect to the frame, each time the soft layer is reattached.

As used herein in the specification and in the claims section that follows, the term "inner face" refers to at least one of: the face of the detachable layer for contacting the surface of the limb segment of the user, and the face of the soft inner layer disposed within the central frame. Thus, in an orthosis in which there is no detachable layer, the term "inner face" refers to the face of the soft inner layer disposed within the central frame.

As used herein in the specification and in the claims section that follows, the term "fully flexible", with respect to the detachable layer, refers to a layer that is not self-supporting.

Central frame **50** is ergonomically configured to at least partially envelop the limb segment, more preferably, to surround at least 2/3 of the circumference of the limb segment, and most preferably, to substantially envelop the limb segment completely. As shown in Figure 2, frame **50** includes at least one circumferentially retaining element pair 12 for tightly enveloping the limb. Retaining element pair **12** includes opposing, flexible and elongated members, such as flexible, elongated member **14** and flexible, elongated member **10c.** Preferably, retaining element pair **12** includes flexible and elongated members that are substantially directly opposite, such as member **14** and member **10d.**

In addition, flexible, elongated members **14, 10a-d** are flexible, and spring-loaded towards the limb segment. Hence, in increasing the diameter of central frame **50,** the pressure applied from within frame **50** must overcome the resistance of the spring-loaded retaining element pair **12,** as well as the resistance of additional flexible and elongated members **10a - 10c.** Consequently, when orthosis **150** is donned by expanding frame **50** around the limb segment, orthosis **150** is tightly held in the desired position by retaining element pair **12** and additional members **10a - 10c.**

Preferably, spring-loaded metal strips **37a, 37b** are disposed around the circumference of frame **50,** to augment the spring-loading action of flexible, elongated members **14, 10a-d,** and to maintain the efficacy of the spring-loading action over the lifetime of orthosis **150.**

Central frame **50** is preferably configured to have one individual elongated member **14** on a first side of retaining element pair **12,** and two to four individual elongated members **10a** to **10d** on the opposing side, more preferably, three elongated members, and most preferably, at least four, as shown in Figures 2 and 3. In this case, frame **50** resembles an open hand where individual retaining members **10a** to **10d** resemble fingers, and individual retaining member **14** resembles a thumb.

While inventive FES orthosis **150** can be designed to have two or more individual retaining member on each side, the inventors have found that having a single, narrow retaining member (such as retaining member **14**) on one of the sides facilitates both the donning process and the doffing process. Preferably, each single, narrow retaining member **14, 10a-d** has a width of 1.0 - 6 cm, more preferably, 2 - 4.5 cm, and most preferably, 2.5 - 3.5 cm. Within these width ranges, this single, narrow retaining member is wide enough to grip the limb segment, and narrow enough to enable facile donning of the orthotic device. With respect to the height of the orthotic device, the single, narrow retaining member has a width W within a range of 8 - 60% of the height of the orthotic device, more preferably, 10 - 35%, and most preferably, 15 - 30%. Above these ranges, central frame **50** acts in a more rigid fashion during the donning process and the doffing process.

Disposed between retaining members **10a** to **10d** are gaps **18a** to **18c,** as shown in Figure 2. Gaps **18a** to **18c** enable elongated members **10a - 10d** to conform in substantially independent fashion to the contours of the limb segment, both when the limb segment is static and dynamic. It should be emphasized that various limb segments exhibit large profile changes, especially during articulations of the neighboring joints, along with activation of the muscles of the particular limb segment.

Thus, the above-described arrangement enables a superior enveloping of the limb segment by frame **50,** and serves to effectively disperse the pressure and strains on the limb tissue, retaining thereby the natural profile and geometry of the limb tissue and muscles. The dispersion of pressures via flexible members **10a** to **10d** also enables an orthosis **150** of a particular diameter and contour to accommodate a wide variety of limb diameters and profiles.

Significantly, the open-hand shape of central semi-rigid frame **50** also allows the limb to be firmly gripped and retained in exact position by FES orthosis **150** during donning of the device, until final locking of orthosis **150,** which will be described hereinbelow.

When FES orthosis **150** is donned on a leg, the above-described arrangement is particularly suitable for enabling the orthosis to adapt to anatomical changes with time, as well as to changes due to the contraction and expansion of the muscles during walking, while maintaining the stimulation electrode in accurate position against the contact points on the leg segment, and with even pressure.

Figure 3A is a perspective side view of central frame **50,** revealing a flexible leaf or plate **27,** integral to frame **50,** for supporting the stimulator unit housing or cradle **30** (not shown). Flexible leaf **27** is preferably attached to frame **50** solely on one side (or on a portion of one side), such that pressure exerted on stimulation unit **46** (not shown) is substantially absorbed solely by flexible leaf **27.** This enables the surface electrical stimulation electrodes to maintain a fixed position, and with substantially even pressure, against a pre-determined position on the surface of the limb segment, even when stimulation unit **46** is knocked, pushed, or pulled. In Figure 3A, flexible leaf **27** is connected to frame **50** solely by two narrow necks **27a, b,** so as to minimize pressures and strains on frame **50** due to stimulation unit **46.**

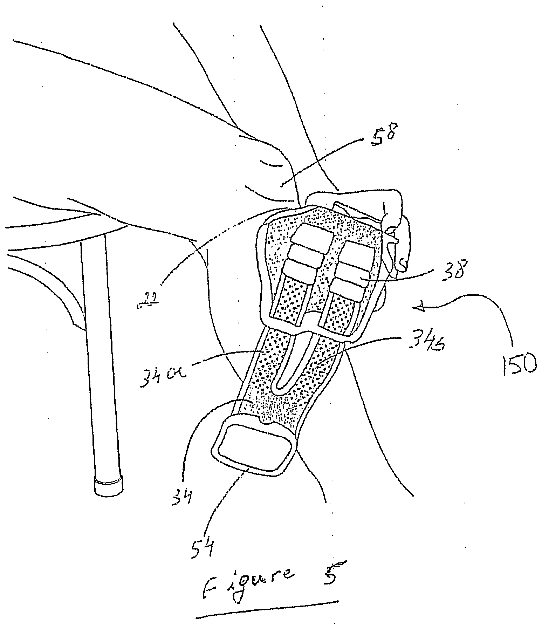

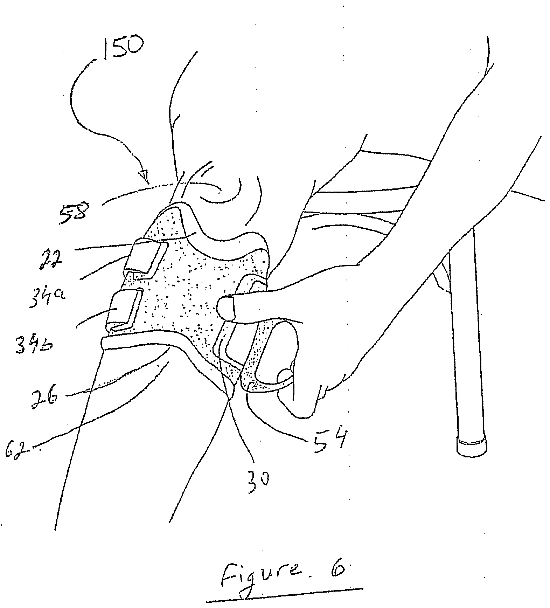

When suitably positioned below the knee, (see also Figures 5 and 6), orthosis **150** provides electrical stimulation to the contact points overlying the Peroneal nerve and the Tibialis Anterior, Peroneus Longus, Extensor Digitorum Longus or extensor Hallucis Longus muscles, so as to modulate the gait. To guide the positioning, central frame **50** preferably includes an upper locator **22,** and a lower locator **26.** Upper locator **22** preferably has the form of a three-dimensional inverted arch, contoured, so as to conform to the inferior border of the tuberosity of the patella and to the characteristic anatomical recesses on each side thereof. The edge of the device is made of elastomeric material to provide comfort when positioned on the limb, and to improve the stability of FES orthosis **150** on the limb segment.

Upper locator **22** includes a molding extending from semi-rigid frame **50** so as to abut the inferior border of the patella, while lower locator **26** is designed to conform to the characteristic anatomical shape of the inferior surface of the tibial crest. When donning FES orthosis **150** on the leg, locator **22** assists both in the accurate longitudinal placement of orthosis **150** along the long axis of the lower leg segment, and in the rotational orientation about the long axis of the leg segment, as will be described in greater detail hereinbelow. Locator **26** assists in the rotational orientation about the long axis of the leg segment.

After exact positioning of FES orthosis **150** on the stimulation points has been achieved, orthosis **150** is firmly secured and locked on to the limb segment by a robust fastening arrangement **34** (shown in Figures 1 and 3), which is firmly associated at a first end, with central frame **50,** and ends in a handle **54** at the opposite end. Fastening arrangement **34** further includes substantially parallel, elastic modular straps **34a** and **34b,** connecting between the first end and handle **54.** Elastic modular straps **34a** and **34b** are designed such that during donning, straps **34a** and **34b** wrap circumferentially around the limb segment, to tightly lock FES orthosis **150** in place around the segment. The locking may be effected by fastening handle **54** to stimulator unit housing or cradle **30.** Various exemplary alternatives for this fastening are provided hereinbelow.

Each of elastic straps **34a** and **34b** is equipped with an adjustment buckle **38** (see Figure 5), so as to allow different degrees of tightening according to the contour of the leg segment and according to the needs of individual patients.

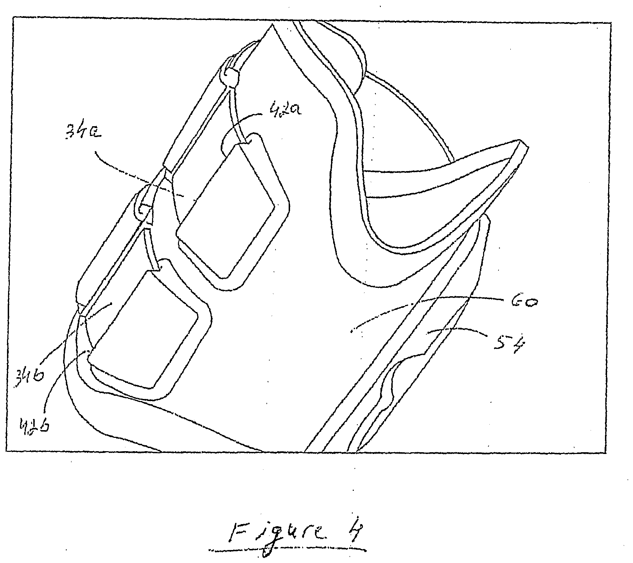

As previously mentioned, central frame **50** is covered by an external layer **60,** which is made of a soft, preferably aesthetic material. Elastic straps **34a** and **34b** emerge through openings **42a** and **42b** in external layer **60,** as shown in Figure 4. This configuration eliminates or greatly reduces the distortion of central frame **50** during the securing of orthosis **150** to the leg.

Handle **54** is preferably made of an elastomeric material that imparts a flexible nature to handle **54.** Preferably, handle **54** has a generally loop-like shape (hollow, with a rectangular or oval perimeter), so as to fit around stimulator cradle **30,** or around any other connecting point or protuberance extending from frame **50,** thereby securely locking orthosis **150** in place over the limb segment. Once positioned around stimulator cradle **30,** handle **54** helps to protect stimulation unit **46** around the sides thereof, thereby providing stimulator cradle **30** and stimulation unit **46** with a smooth and unobtrusive profile. This is particularly important because knocks and pressures delivered to the surfaces of stimulation units of the prior art, in addition to being unpleasant for the user, can compromise the substantially even pressure applied by the surface electrode to the surface of the limb segment. The streamlined profile also facilitates rolling a pant leg past orthosis **150.**

Fastening arrangement **34** is modular, can be easily detached from orthosis **150,** and can be manufactured in several sizes.

Figures 5 and 6 illustrate the donning -- and subsequent locking -- of FES orthosis **150** on an impaired leg. For positioning orthosis **150,** while seated, it is preferable for the user to partially extend the lower leg, as shown in Figure 5, such that the protuberance of the patella **58** is clearly defined. Subsequently, orthosis **150** is placed on the leg, such that upper locator **22** is juxtaposed against the lower facing of patella **58.** Lower locator **26** should then be centered around tibial crest **62.** Orthosis **150** grips the leg gently, but firmly enough to keep orthosis **150** in place, even if the user releases his grip as in the case, inter alia, of hemiplegic users. Subsequently, the securing of orthosis **150** is completed by means of fastening arrangement **34.**

An alternative donning procedure is to place FES orthosis **150** along the tibial crest **62,** a few centimeters below patella **58,** and then to gently slide orthosis **150** up the calf until upper locator **22** abuts against patella **58.** After orthosis **150** grips the leg segment so as to retain the desired position, locking is achieved by grasping handle **54** with the fingers of the opposite hand from the leg with orthosis **150.** The thumb is placed on cradle **30,** to hold orthosis **150** in place, and preventing sliding down or rotation, while the hand is closed so that the loop of handle **54** fits snugly around cradle **30.** Alternatively, four fingers of the hand opposite to the leg with orthosis **150** are slipped through handle **54,** grasping the handle close to cradle **30.** The fingers then curl onto the attachment point of cradle **30** and lever handle **54** into place while allowing handle **54** to slip off the fingers. Once handle **54** is locked in place around cradle **30,** the tension of fastening arrangement **34** firmly holds orthosis **150** around the limb segment of the user, even during aggressive movement of the limb.

FES orthosis **150** is doffed by releasing handle **54** from cradle **30** and pulling orthosis **150** away from the leg segment. It should be emphasized that both donning and doffing may easily be performed, unassisted and using a single hand, by hemiplegic patients.

Members **10a** to **10d** and member **14** of central frame **50** are preferably made of a polymeric material that provides flexibility and spring-like characteristics to orthosis **150.** This combination of structure and materials provide the following features to orthosis **150:**
1. Facile placement on the leg using a single hand.
2. Spring-loaded retention or gripping of orthosis **150** in proper position prior to and during locking orthosis **150** in position. Both the facile placement of the orthosis and the self-retaining grip of the orthosis on the leg enable patients suffering from an impaired hand to effectively don the device.
3. Accurate locating and relocating of the stimulating electrode assemblies.
4. After locking of orthosis **150** on the limb, the structure and materials of these members prevent random movement and migration of orthosis **150,** even during limb extensions, flexions and gait.
5. Uniform dispersion of the pressure and strains to the limb tissue, thus retaining the natural profile and geometry of the limb tissue and muscle.
6. Uniform pressure of the electrodes against the surface of the limb segment.

Referring now to Figures 1, 3 and 7, stimulator unit **46** is associated with orthosis **150** by means of cradle **30,** which is a receptacle integrated onto the external surface of central frame **50.**

Cradle **30** is advantageously designed to have concave edges for receiving a thumb during the donning procedure. Placing the thumb on the edges of cradle **30** enables the formation of a counter-force for urging handle **54** towards cradle **30.** The backside of cradle **30** has a concavity similar to that of central frame **50,** such that the overall contour can adapt to the contour of the limb segment.

Stimulator unit **46** is small and has a thin profile, so as to minimally protrude from the surface of orthosis **150,** and is lightweight, to avoid superfluous weight on the impaired leg.

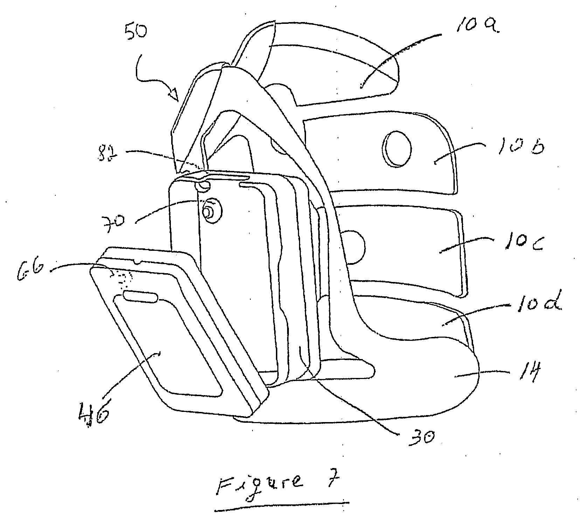

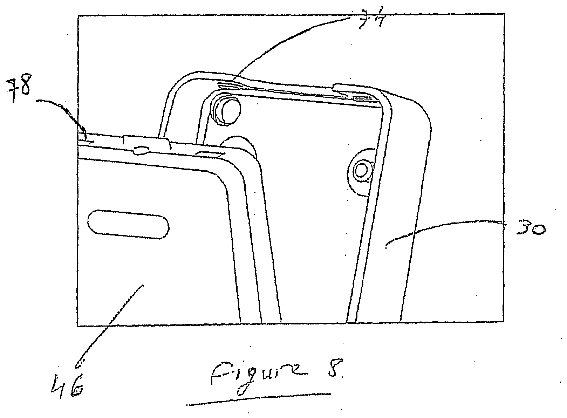

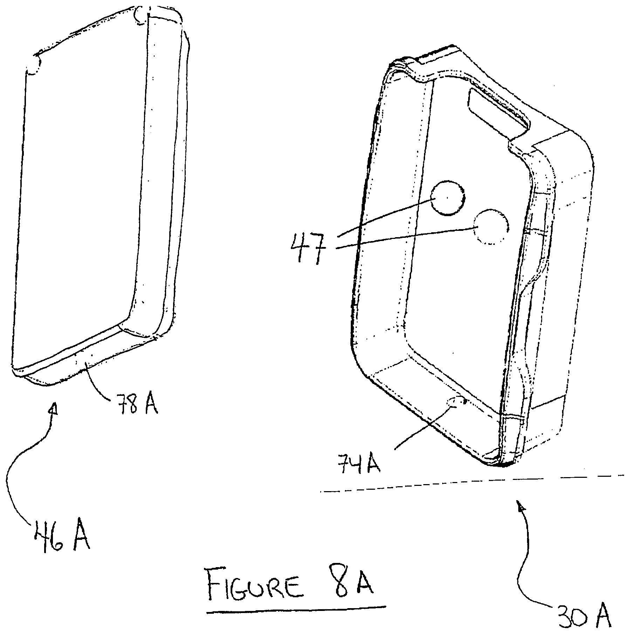

Stimulator unit **46,** as shown in Figures 7 and 8, is electrically connected to cradle **30** by connectors **66** and **70** disposed on stimulator unit **46** and on cradle **30,** respectively. In one preferred embodiment, these connectors are complementary connectors. As shown, connector **66** is a complementary female snap, and connector **70** is a corresponding male snap. Stimulator unit **46** is mechanically connected to cradle **30** by complementary mechanical connectors **74** and **78.** In the embodiment shown in Figure 8, these connectors are female latch receiver **74** disposed on an edge of cradle **30,** and female latch receiver **78** disposed on an edge of stimulator unit **46.** An additional notch **82** in cradle **30** allows pulling stimulator unit **46** with a finger or thumb to release it from cradle **30.** These connective features enable facile detaching and reattaching of stimulator unit **46** from, or to, cradle **30** using a single hand.

Figure 8A is a schematic perspective view showing a neuroprosthetic stimulator unit 46A and a complementary cradle **30A,** according to a preferred embodiment of the present invention. As in the embodiments shown in Figures 7 and 8 and described hereinabove (with reference to stimulator unit **46** and cradle **30**), stimulator unit **46A** is electrically connected to cradle **30A** by conducting connectors disposed on stimulator unit **46A** and on cradle **30A,** respectively (not shown). The electrical connection to the orthosis is via at least one opening **47** in the back panel of cradle **30A.** Preferably, a complementary connector such as a snap connector is disposed in each opening **47** (see connectors **66** and **70** in Figure 7 and the associated text hereinabove).

Stimulator unit **46A** is mechanically connected to cradle **30A** by complementary mechanical connectors **74A** and **78A** of the pin and recess variety. In Figure 8A, stimulator unit **46A** is equipped with a pin **78A** disposed on a side facing, and cradle **30A** is equipped with an opening **74A** disposed in the respective side facing, such that upon inserting stimulator unit **46A** into cradle **30A,** pin **78A** is introduced into opening **74A.** Once stimulator unit **46A** is snapped into cradle **30A,** pin **78A** is locked into place within opening **74A**, thereby affixing stimulator unit **46A** to the orthosis and ensuring a secure electrical connection between stimulator unit **46A** and the electrodes, even when various mechanical strains are exerted on the orthosis during the course of physical activity.

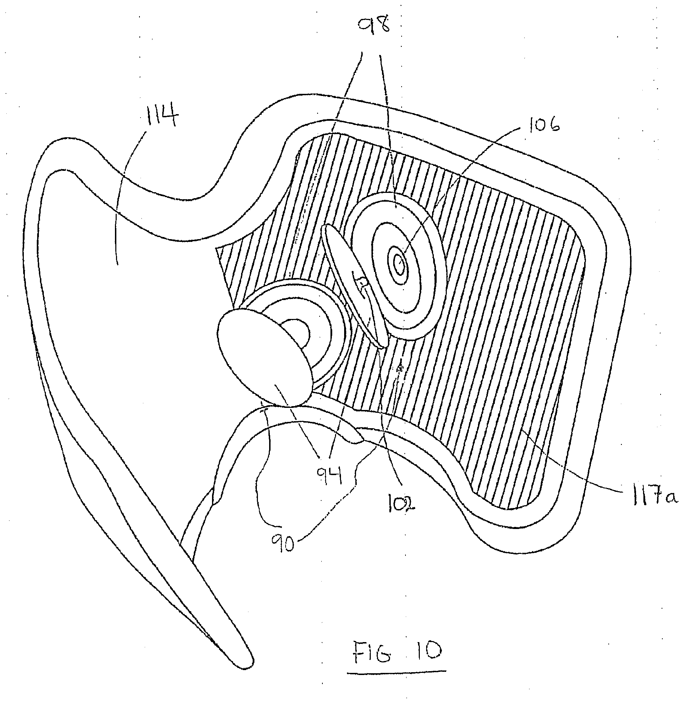

Referring now to Figure 9, Figure 9B and Figure 10, Figure 9 is a perspective view of an electrode assembly **90** of inventive orthosis **150** (not shown). Electrode assembly **90** includes a surface electrode **94,** an electrode base **98,** and complementary conductive connectors **102** and **106.** Electrode assembly **90** further includes a conductive wire **110,** which, at a first end, is mechanically connected to electrode base **98** so as to provide an electrical connection to connector **106.** Conductive wire **110** terminates at a second end with a complementary conductive connector **122,** which mechanically connects electrode assembly **90** to central frame **50** (as shown below in Figure 9B), and electrically connects electrode assembly **90,** via central frame **50,** to stimulator housing **30** (also shown below in Figure 9B).

In the exemplary embodiment shown in Figure 9, complementary connector **102** is a male snap connector associated with surface electrode **94,** while complementary connector **106** is a female snap connector connected to electrode base **98.** Both surface electrode **94** and electrode base **98** are made of flexible materials that enable them to conform to the limb tissues.

Electrode base **98** is preferably concave-shaped, such that the connection between complementary connector **102** of surface electrode **94** and complementary connector **106** of electrode base **98** is recessed within a recess, thereby preventing excessive local pressure of the snaps **102** and **106** on the underlying skin. The concave, 3-dimensional shape of the top surface of electrode base **98** also provides a substantially even contact pressure of surface electrode **94** to the skin.

Surface electrode **94** preferably has a large surface area, with respect to many FES leg devices of the prior art, which serves to assuage discomfort from the electrical stimulation. The surface area of surface electrode **94** is preferably at least about 9 cm², more preferably at least about 12 cm², and even more preferably, at least about 15 cm². In some cases, the surface area of surface electrode **94** is as much as about 20 cm². It is presently preferred that the surface area of surface electrode **94** is within a range of 12 - 20 cm². By sharp contrast, the surface area of each surface electrode of the prior art devices such as the WalkAide® device is less than about 5 - 7 cm².

In contrast to prior art FES leg devices, the electrode separation in the FES orthosis of the present invention is preferably as large as anatomical constraints permit, particularly in the direction along the length of the limb. The distance between electrode centers is at least about 5 cm and the longitudinal separation is at least about 3 cm, and preferably at least 3.5 cm.

Figure 9A is a perspective view of another embodiment of an electrode assembly **90A** of inventive orthosis **150** (not shown). Electrode assembly **90A** includes a surface electrode **94A,** an electrode base **98A,** and a wire **110,** which terminates with complementary connector **122.** In the exemplary embodiment shown in Figure 9A, surface electrode **94A** has first and second faces **95, 97** made of hydrogel or any another conductive, adhesive material known in the art for use in surface electrodes. First face **95** is for flexibly adhering to the skin of the user, while the opposite second face **97** is for adhering to a top face **99** of electrode base **98A.** On top face **99** is disposed a conductive region **106A,** for electrically connecting with second face **97.** Preferably, top face **99** of electrode base **98A** is also provided with a rim **101** for tightly receiving surface electrode **94A**, such that the relative position of electrode **94A** and electrode base **98A** is uniquely defined and determined. The connection of electrode **94A** and electrode base **98A** may be further enhanced by disposing, on top face **99** of electrode base **98A,** patches **103** of hook fasteners (e.g., plastic Velcro® hooks), which surprisingly hold on to the substantially flat, hydrogel surface of second face **97.**

Substantially as described above with respect to Figure 9, conductive wire **110** is mechanically connected to electrode base **98** so as to provide an electrical connection to conductive region **106A**.

Another preferred embodiment of an electrode base **98B** according to the present invention is shown schematically in Figures 9C and 9D. As in previous embodiments, electrode base **98B** is equipped with conductive region **106A**, for electrically connecting with a face of a surface electrode such as surface electrode **94A** (shown in Figure 9A). Electrode base **98B** is also equipped with a cover such as ring-shaped cover **107.** Cover **107** is preferably connected to electrode base **98B** by means of a hinge **109,** which in one preferred embodiment, is obtained simply by attaching (e.g., by ultrasonic welding) cover **107** to electrode base **98B.** Alternatively, base **98B** and cover **107** can be manufactured as one piece, which is subsequently folded in the middle to create hinge **109.**

After insertion of the surface electrode between electrode base **98B** and cover **107,** cover **107** is pressed down in the direction of base **98B.** A tongue **111** disposed roughly opposite hinge **109** facilitates opening and closing of cover **107.**

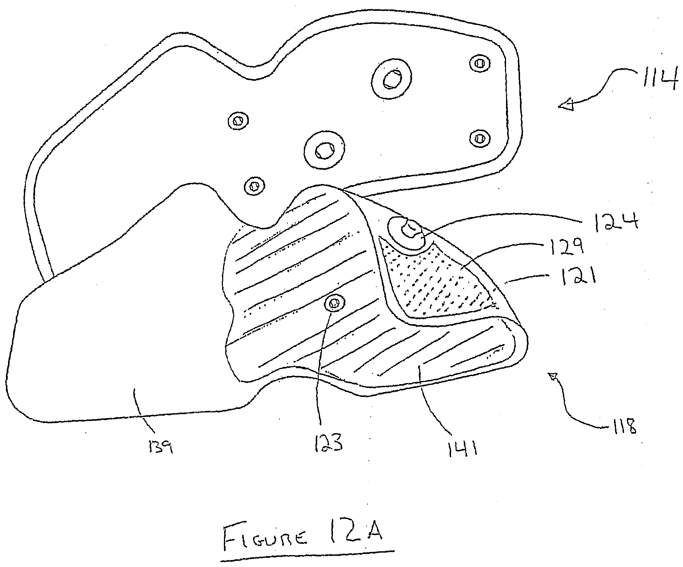

Preferably, a bottom surface of cover **107** and tongue **111** is at least partially covered with complementary connectors **113,** preferably hook and loop fasteners, for connecting to complementary connectors **117a** on internal layer **114,** shown in Figures 9B and Figure 10. Alternatively, complementary connectors **113** may be connected to complementary connectors **141** on a top facing **139** of a detachable layer **118** shown below in Figures 12A-12B.

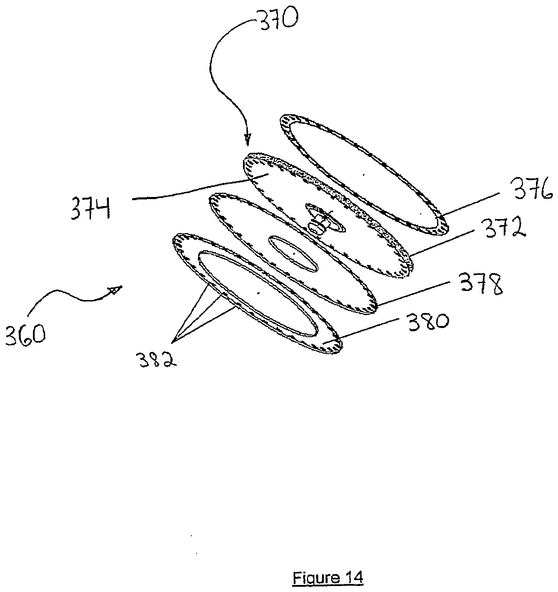

Cover **107** is adapted such that the perimeter of the surface electrode is held firmly in place. Also, cover **107** protects the edges of the hydrogel layer (see, by way of example, hydrogel layer **372** in Figure 14 hereinbelow) to prevent or greatly reduce disintegration of the edges of the hydrogel layer.

A top view of cover **107** and electrode base **98B** is provided in Figure 9D. A dotted line **127** represents the circumference of electrode base **98B.**

Electrode base **98B** may advantageously have an edge or perimeter of a different color or texture than the rest of base **98B,** so as to direct the user in affixing the surface electrode in the proper position on base **98B.**

The electrode may also be attached directly to the orthosis, e.g., by attaching hook and loop fasteners to the face of the electrode facing the orthosis.

The relationship between electrode assembly **90** and internal soft layer **114,** shown in Figure 10, is also relevant for the alternative embodiment presented in Figure 9A and described hereinabove. Electrode base **98** is attached to internal layer **114** by complementary connectors **117** and **117a,** preferably hook and loop fasteners such as Velcro®, which are best seen in Figure 9B.

The electrical connections are made as follows: complementary connector **122** connects to a complementary connector **123,** which is disposed in a fixed position on internal layer **114** or on central frame **50.** Electrical stimulation is achieved by directing a current from stimulator unit **46** (not shown), via connector **70** disposed on housing **30,** through frame **50** and internal layer **114,** to connector **123,** and ultimately, to electrode assembly **90A** and stimulator housing **30** with connector **123.**

In the exemplary embodiments shown, complementary connector **122** is a conductive male snap connector, and complementary connector **123** is a conductive female snap connector.

It will be appreciated that the design of electrode base **98** and the design of connectors **122** and **123** enable a wide range of positioning of each electrode assembly **90.** Unlike various prior art devices, the position of electrode **94** is not limited to a position in which electrode **94** physically touches a conductive element fixed in the body of the orthosis (such as connector **123**). Rather, the position of electrode **94** is substantially decoupled with respect to such fixed conductive elements. Wire **110** should be long enough to enable the full range of positioning.

In addition, the above-described design enables the clinician to exactly define the position of each electrode assembly **90** in a first step, and then, in a second step, to attach the assembly to FES orthosis **150** by means of complementary connectors **122** and **123.** Thus, during the initial setup procedure, the clinician needs to position solely the surface electrodes or electrode assemblies (e.g., electrode assembly **90A**) -- and not the entire orthosis **150** -- on the stimulation points of the muscles to be activated. In order to define the exact position, the clinician activates the electrodes, which can electrically be connected directly to stimulator unit **46.** Subsequently, the clinician connects each surface electrode **94** to the respective electrode base **98** prior to donning orthosis **150** on the leg and prior to attaching each electrode base **98** to internal layer **114** or to central frame **50.** Once orthosis **150** has been donned, each electrode base **98** attaches, by means of complementary connectors **117** and **117a** (shown in Figure 9B), to internal layer **114** of orthosis **150.**

Stimulation by means of the two electrode assemblies **90,** illustrated in Figure 10, produces dorsi-flexion of the foot. To ensure electrical contact between surface electrode **94** and the skin surface, surface electrode **94** may be smeared by conductive gel or hydrogel or covered by a cloth pad that has been soaked in a conductive liquid such as water. The face of surface electrode **94** for contacting the skin may advantageously be a flexible hydrogel layer.

Electrode assemblies **90** conform to the three-dimensional shape of the underlying limb, and adapt their shape during limb articulations and muscle contractions. Electrode base **98** and surface electrode **94,** which are essentially elastic, lie pressed between internal soft layer **114** and the skin surface, such that a minimal resistance is supplied to the changing limb geometry.

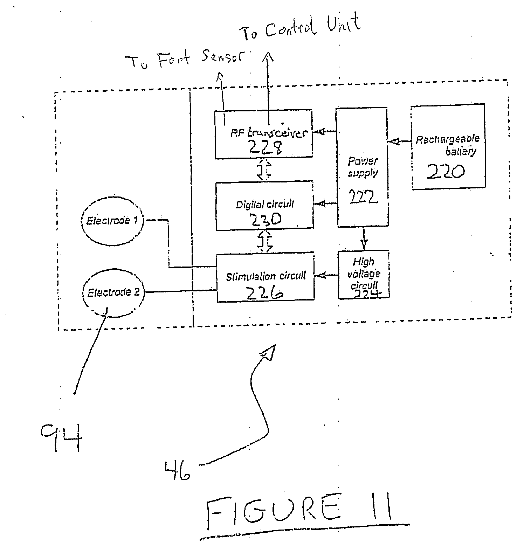

Referring now to Figure 11, stimulator unit **46** is electrically connected with at least one stimulation electrode, such as surface electrode **94.** Stimulator unit **46** is preferably powered by a rechargeable battery **220** that is electrically connected to an internal power supply **222.** Power supply **222** supplies power to a high-voltage circuit **224** feeding into stimulation circuit **226.** Preferably, stimulator unit **46** communicates with a stimulator control unit and/or a limb or motion sensor (described hereinbelow) by means of a radio frequency (RF) transceiver **228.** In a presently-preferred embodiment, RF transceiver **228** transmits signals to a control unit under normal circumstances. However, RF transceiver **228** transmits signals directly to the foot sensor device (described hereinbelow), under special, pre-defined circumstances, such as:
1. In the RF registration process (when the present sensor and/or stimulation unit are replaced by new ones, and are registered to each other and to the control unit), and
2. When there is a long period of sensor inactivity, the sensor requests the authorization from the stimulation unit to enter a sleep mode. The stimulation unit then transmits a signal to the sensor, indicating authorization granted or authorization denied.

Power supply **222** supplies power at a lower voltage to RF transceiver **228,** and to a digital circuit **230** interfacing between RF transceiver **228** and stimulation circuit **226.**

As used herein in the specification and in the claims section that follows, the term "radio frequency" refers to electromagnetic waves, preferably having a frequency within the radio frequency range. The currently preferred range is 2400-2483.5 GHz.

During operation, the battery-operated control unit maintains two-way communication with stimulator unit **46.**

The control unit enables to switch on the device, select the operating mode of the device (gait mode, training mode, or returning to standby mode), and adjust the intensity of stimulation and volume of an audio alert.

Additionally, each of the system components (FES orthosis **150,** control unit and foot sensor) is preferably represented by graphic icons on the control unit. LEDs of different colors emit light under the relevant icon so as to indicate attention-requiring events such as low battery or a malfunction of any individual component. Other LEDs indicate stimulating or resting, stimulation intensity, or training mode.

The control unit preferably has an audio alert that produces an audio signal so as to alert the user when: the system is first switched on, a button has been pressed, a mode has been selected, the battery is low, radio communication between components of the device is lost, or, other faults requiring the attention of the user have occurred. The control unit may be waist-mounted, hung by means of a neck strap, or mounted by an in-pocket hold.

The foot sensor unit may be any of various foot sensor units known in the art, including a force sensor disposed underneath the foot of a user, a tilt sensor, etc.

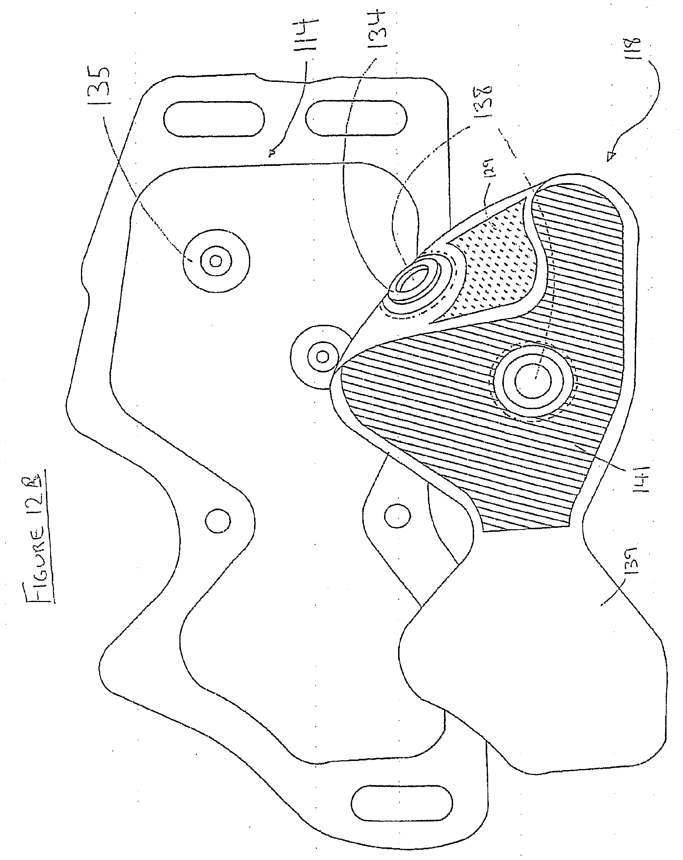

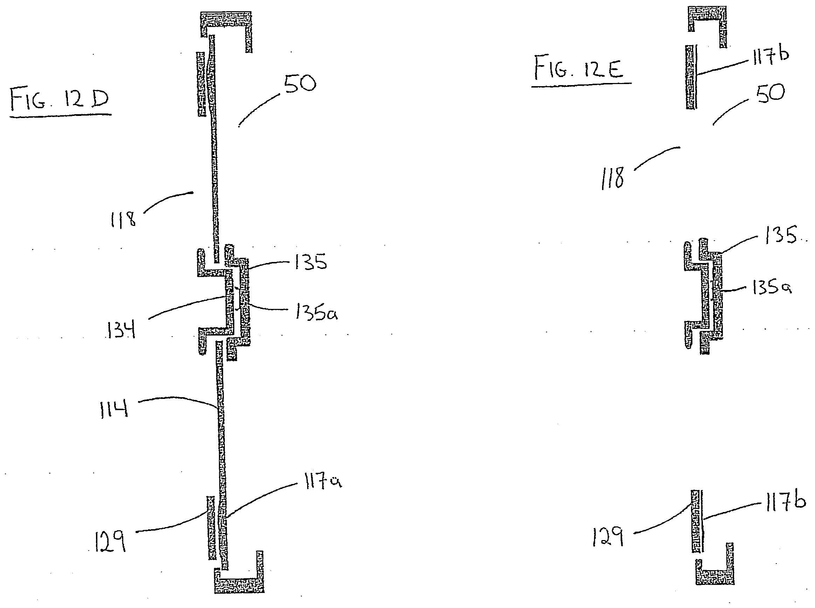

Figures 12A - 12F schematically depict another aspect of the present invention. Figures 12A and 12B show a front view of a personal panel or detachable layer or liner **118** for attaching to the orthosis, either to internal soft layer **114,** as shown in a schematic side cross-sectional view of a slice of the orthosis in Figure 12D, or directly to frame **50,** as shown in the identical view in Figure 12E.

Detachable layer **118** preferably has, substantially, the shape of internal soft layer **114.** Detachable layer **118** is soft, so as to provide a comfortable feel on the skin surface of the user, but is provided with sufficient rigidity to maintain the alignment of each electrode assembly **90** and to make it easier to position detachable layer **118** to the orthosis. Suitable materials for detachable layer **118** include various non-woven materials such as Nordenia™ or Namliong™ loop. Detachable layer **118** preferably has a first or top facing **139** having a plurality of complementary connectors **141,** such as loop fasteners, disposed on the surface.

Detachable layer **118** may include the compatible electrical connectors of internal layer **114.** Thus, detachable layer **118** may include male snap connectors **124** on a second or back surface **121** facing FES orthosis **150** and complementary connectors such as female snap connector **123** on top facing **139,** for receiving complementary connector **122** (shown in Figures 9, 9A, and 9B) and described hereinabove. In this arrangement, detachable layer **118** is attached by the clinician to internal layer **114,** or to another portion of the orthosis, such as frame **50,** by means of snap connectors **124.** This provides the requisite electrical contact, and ensures accurate, singular, repeatable positioning and fixing of detachable layer **118** onto internal layer **114** and/or with respect to frame **50.** Alternatively, back surface **121** has at least one patch **129** of hook or loop connectors that fix detachable layer **118** to internal layer **114.** In Figure 12A, by way of example, patch **129** includes hook connectors.

In another preferred embodiment, shown in Figure 12B, detachable layer **118** includes holes **138,** which are advantageously disposed to line up between complementary male connectors **122** of electrode assembly **90** and complementary female connectors **123** on internal layer **114,** such that connector **122** connects electrode assembly **90** - mechanically and electrically, to stimulator unit housing **30.**

It must be emphasized that the repositioning of detachable layer **118** on FES orthosis **150,** for a particular individual, restores accurate, substantially repeatable positioning of the electrode assemblies **90,** even when FES orthosis **150** is a standard, universal unit that has not been adapted to the needs of that individual. Thus, in sharp contrast to prior art devices, inventive detachable layer **118** enables the clinician to use a single FES orthosis **150** for treating many users in the clinic. An individual detachable layer **118** is dedicated for a particular user. Each particular user undergoes a pre-fitting session with the clinician, so as to customize the electrode positioning for the needs of that user. Subsequently, detachable layer **118** is used repeatedly in future clinical sessions of the user. The clinician, who works with many users each day, simply removes the pre-fitted internal soft layer belonging to the previous user and attaches the pre-fitted internal soft layer belonging to the current user. The detachable layer also serves as a hygienic layer where the device is shared between users.

The replacement of the detachable layer **118,** with no need to relocate the electrode assemblies **90** for each patient, enables the clinician to provide efficient, effective treatment to numerous orthosis users in a short period of time. Detachable layer **118** is preferably disposable. Alternatively, detachable layer **118** is washable.

According to another preferred embodiment of the present invention, shown schematically in Figure 12B, a hollow snap connector **134,** which is preferably an annular or oval hollow snap connector, is associated with a perimeter of hole **138** of detachable layer **118.** Hollow snap connector **134** accurately and reliably snaps into a corresponding hole or recess **135,** thus ensuring that detachable layer **118** is tightly attached to internal layer **114,** and is exactly positioned in the same location upon each application. In addition, hollow snap connector **134** also serves for mechanically attaching complementary connector **122** of electrode assembly **90** to internal layer **114,** via detachable layer **118.**

Hollow snap connector **134** can be made of a plastic, flexible material such as PVC.

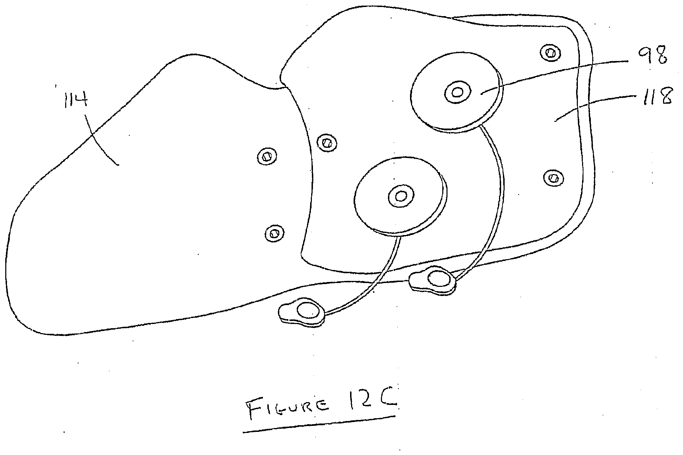

It should be appreciated that detachable layer or liner **118** may also have a shape other than the contour of internal layer **114.** For example, detachable layer **118** may cover only a portion of the surface of orthosis **150,** as shown in Figure 12C. Figure 12C also shows electrode base **98** affixed to detachable layer **118.**

Figures 12D and 12E, described briefly hereinabove, show some of the main electrical and mechanical connections between detachable layer **118** and other components of the orthosis, wherein, in Figure 12D, detachable layer **118** is attached to central frame **50** and to internal layer **114,** and wherein, in Figure 12E, layer **118** is directly attached to central frame **50.**

In Figure 12E, a complementary connector such as hollow snap connector **134,** is disposed on detachable layer **118.** Hollow snap connector **134** accurately and reliably snaps into a corresponding hole or recess **135** disposed in central frame **50,** so as to directly connect detachable layer **118** to central frame **50.** This also ensures that detachable layer **118** is tightly associated with internal layer **114,** and that detachable layer **118** is exactly positioned, with respect to central frame **50,** in the same location upon each application.

As described hereinabove with reference to Figure 12A, back surface **121** of detachable layer **118** preferably has at least one area or patch **129** of complementary connectors (e.g., hook or loop connectors) that fix detachable layer **118** to internal layer **114** (via complementary connectors **117a**).

The description of the attachments of Figure 12D largely applies to those of Figure 12E. In Figure 12E, however, detachable layer **118** is juxtaposed against central frame **50,** consequently, patch **129** of complementary connectors on back surface **121** is configured to fix detachable layer **118** to central frame **50** by means of complementary connectors **117b.**

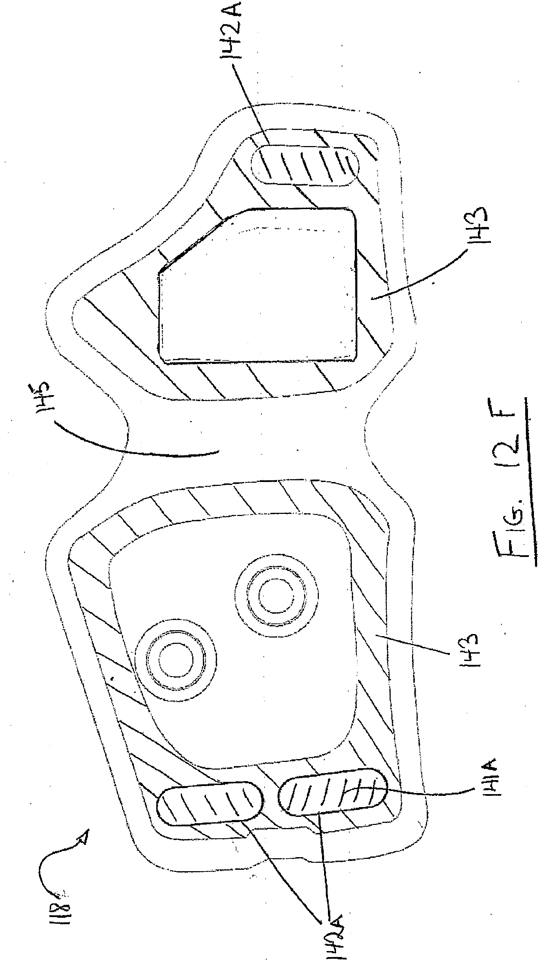

In another preferred embodiment, inventive detachable layer **118** is stiffened by a supporting region or frame. A first side of detachable layer **118** is schematically provided in Figure 12F. Detachable layer **118** is largely similar or even substantially identical to detachable layer **118** described hereinabove, and can be connected to an internal layer of the orthosis, or to another portion of the orthosis, in similar or identical fashion. Preferably, the first side of detachable layer **118** has a plurality of complementary connectors **141A,** preferably, hook or loop fasteners, disposed thereon. Typically, complementary connectors **141A** are disposed within distinct fields or regions **142A** on detachable layer **118.**

At least one supporting region **143** of a stiffening or supporting material are attached to detachable layer **118,** in order to temper the flexibility of detachable layer **118.** The disposition of supporting region **143** on detachable layer **118** simplifies the assembly process of detachable layer **118** on the orthosis and also ensures that detachable layer **118** has a smooth, unwrinkled surface. Polypropylene is one suitable material for region **143.** The attachment of region **143** to underlying detachable layer **118** may be performed by sewing, gluing, or other means known to those skilled in the art.

As shown in Figure 12F, an area **145** on detachable layer **118** is preferably free of supporting material, so as to enable detachable layer **118** to flexibly bend around the leg of the user without undue mechanical stress, and to allow at least part of detachable layer 118 to "breathe", thereby avoiding excessive perspiration.

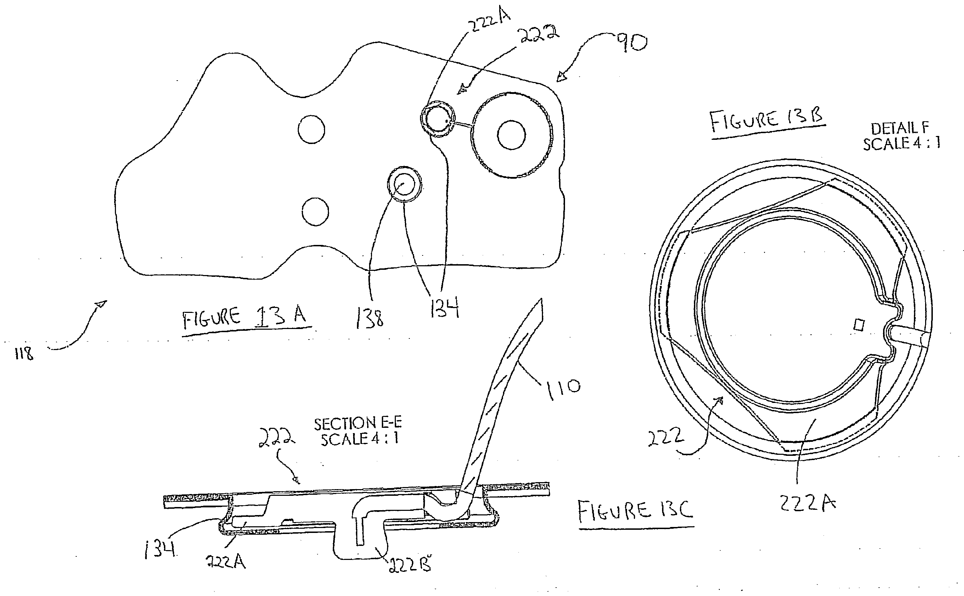

With reference to Figures 13A-C, and Figures 12D-E, Figure 13A is a schematic perspective view of electrode assembly **90** and detachable layer **118,** according to another embodiment of the present invention. As above, detachable layer **118** is equipped with hollow snap connector **134,** which, as shown and described hereinabove, is configured to accurately and reliably snap into a corresponding complementary recess **135** disposed in central frame **50,** so as to directly connect detachable layer **118** to central frame **50.**

A complementary conductive connector **222** of electrode assembly **90** has a conductive snap **222B** (shown in Figure 13C) designed to pass through a hole such as hole **138** of hollow snap connector **134,** and into a complementary area **135a** of complementary recess **135** so as to both mechanically and electrically connect electrode assembly **90** to central frame **50.** The perimeter of complementary conductive connector **222** has fins or protruding elements **222A,** for snap connecting into the complementary contour of hollow snap connector **134.**

Thus, hollow snap connector **134** mechanically connects to complementary recess **135,** complementary conductive connector **222** mechanically connects to hollow snap connector **134** by means of protruding elements **222A,** and complementary conductive connector **222** electrically connects to complementary recess **135** by means of conductive snap **222B.**

It should be emphasized that complementary conductive connector **222** can be directly attached to complementary recess **135** when no personal panel is used in the orthosis.

Another aspect of the present invention relates to the electrodes of the orthosis. Hydrogel electrodes are commonly used for electrical stimulation applications, as well as for other applications. The conductive hydrogel layer is used to ensure and improve the electrical contact between the electrode and the skin surface.

Often, after a period of time of using the electrode, the hydrogel layer tends to peel off from the electrode edges surface. This is due, apparently, to the pull and shear stress acting on the hydrogel layer, and to the weakening of the hydrogel layer attachment to the underlying surface thereof. This problem is especially prevalent when a self-adhesive hydrogel electrode is used, and when the electrodes are positioned under an overlying layer, such as when positioned inside an orthosis or a sleeve.

According to the present invention, in order to eliminate the disintegration of the edges of the hydrogel layer from the underlying layer, the hydrogel layer is mechanically attached to the underlying electrode surface. Referring now to an exemplary embodiment provided in Figure 14, the inventive electrode assembly **360** has a disk-shaped electrode **370** having a hydrogel layer **372** and an underlying electrode surface layer **374.** Mechanical attachment is achieved by providing an additional, thin layer or film, such as first film ring **376,** disposing hydrogel layer **372** between underlying layer **374** and film ring **376,** and attaching or bonding first film ring **376** to underlying layer **374.** Electrode **370** is adapted such that hydrogel layer **372** is for facing, and adhering to, the skin surface of the user, while underlying layer **374** is for facing the orthosis. The mechanical attachment can be achieved by an ultrasonic process, or by other processes known to those skilled in the art, including, but not limited to, sewing, gluing, etc. In this manner, hydrogel layer **372** is confined between film ring **376** and underlying electrode surface layer **374.**

Alternatively or additionally, first film ring **376** can be attached to an additional thin layer or film, such as second film ring **378.** The attachment to second film ring **378** may improve the connection between first film ring **376** and electrode **370.** The attachment of first film ring **376** to second film ring **378** may be performed by ultrasonic welding. It must be emphasized that is often not possible to make an ultrasonic weld between typical electrode materials and an adjacent surface.

An additional layer **380** having attachment points or an attachment mechanism **382** may also be connected to electrode **370,** on the side of underlying electrode surface layer **374,** so as to improve the attachment of the electrode to the electrode base or to any underlying layer. In the exemplary embodiment provided in Figure 14, additional layer **380** is a ring, and attachment mechanism **382** is of the hook-and-loop (e.g., Velcro®) variety. Such an attachment mechanism is instrumental in preventing an electrode from sticking to the skin and becoming detached from the electrode base during removal of the base (or sleeve) from the skin surface. The hook-and-loop material on additional layer **380** may also improve the attachment of second film ring **378** to electrode **370.**

In a presently preferred embodiment, first film ring **376** and second film ring **378** each have a thickness of about 50-200 microns. A thickness of about 125 - 175 microns is presently preferred. The presently preferred material of construction is clear polypropylene.

Another aspect of the present invention is a foot sensor device for gait enhancement, having a fastening unit, for securing the electronic communication unit to the shoe of the user, in a substantially fixed position, during gait of the user.

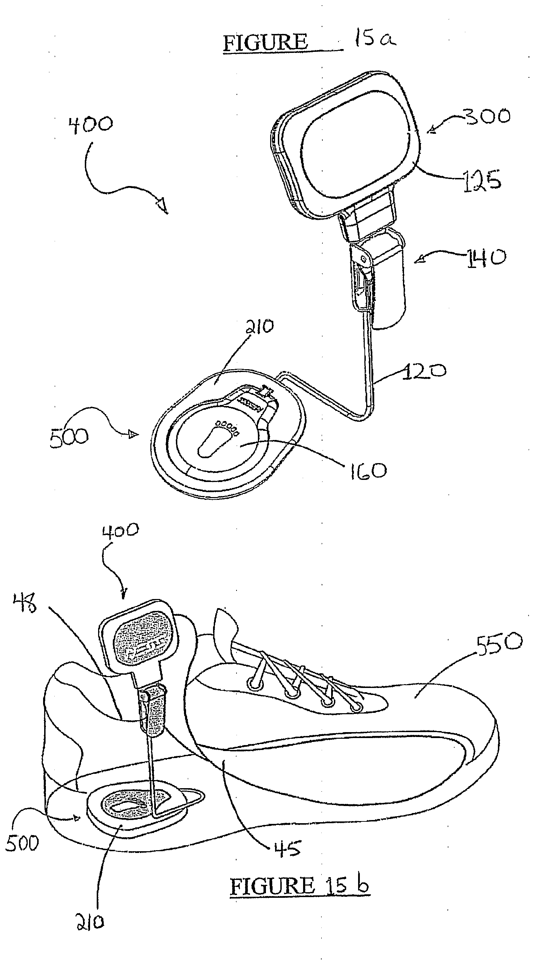

Figure 15a is a perspective view of the inventive foot sensor device **400** for a gait-enhancing orthosis. Foot sensor device **400** includes a sensor unit **500** having a sensor casing **210,** in which is disposed a sensor element **160,** a communication unit **300** enclosed by housing **125,** and wiring (wire or cable) **120** for mechanically and electrically connecting sensor element **160** and communication unit **300.** Foot sensor device **400** further includes a fastening unit such as clamp unit **140,** rigidly or at least semi-rigidly attached to communication unit **300,** for attaching communication unit **300** to the user's shoe, and more typically, to the rim of the user's shoe (see Figure 15b hereinbelow). It should be stressed that sensor element **160** of inventive foot sensor device **400,** in contrast to RF-based foot sensor devices of the prior art, is substantially mechanically independent of communication unit **300.** The advantages of this attribute are elaborated hereinbelow.

As used herein in the specification and in the claims section that follows, the term "substantially mechanically independent" refers to two components, electrically and mechanically connected, and physically set apart from one another, the components having substantially no constraint when moved towards one another. A typical example of such substantially mechanically independent components is communication unit **300** and sensor casing **210,** connected by a flexible conductive wire.

Generally in the prior art, the foot pressure switch, or the sensor, is either permanently disposed in the shoe, or disposed in a small pouch of a sock, as in the above-described ActiGait® system. As shown in Figure 15b, by sharp contrast, sensor unit **500** is preferably placed under an inner sole **45** of a shoe **550,** beneath the heel area. Sensor casing **210** can be removed or repositioned with facility, by lifting up or removing inner sole **45.**

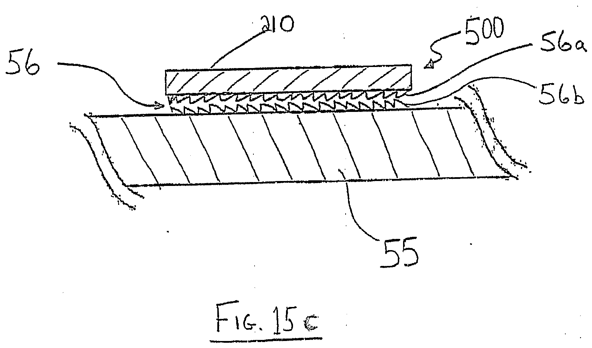

As shown in a cross-sectional view in Figure 15c, sensor unit **500** may advantageously secured to the floor **55** of the shoe by a hook and loop system **56** such as Velcro®. By way of example, hooks **56a** of hook and loop system **56** may be attached to sensor casing **210,** and loops **56b** may be attached to floor **55.** In particular cases, sensor casing **210** may also be positioned under the forefoot area of the shoe.

Casing **210** is configured to protect the inner sensor element against mechanical stress and wetness, and is made of two flexible layers, selected such that the upper layer is more flexible than the lower layer. An additional piece of sponge-like material lies between the internal layer and the sensor, providing additional protection to the sensor. The two layers of sensor casing **210** are attached by various means such as ultrasonic welding, RF welding, gluing, heat welding, or pins. Casing **210** is connected to communication unit **300** by means of wiring **120,** which is long enough to enable attachment of communication unit **300** to a rim **48** of shoe **550,** irrespective of the height of the shoe rim. It should be noted that when the shoe is not worn, foot sensor device **400** can be left attached to shoe **550,** such that the user is not challenged with an additional, cumbersome, and unintuitive doffing action, and in subsequent use, a similarly challenging donning action.

The positioning of communication unit **300** above the rim of shoe **550** protects communication unit **300** from being banged and rubbed by external objects, and from exposure to dirt and moisture. Such positioning may also serve to keep communication unit **300** hidden under the trousers, which is aesthetically desirable and, perhaps more importantly, protects communication unit **300** from being caught or bumped during gait.

Moreover, in foot sensor device **400** of the present invention, the height of communication unit **300** on the leg of the user is substantially fixed with the height of the shoe rim. In the above-referenced design patent to Haugland, et al., by sharp contrast, the distance between the communication unit and the sensor unit is determined by the semi-rigid spine connecting therebetween, such that the height of the communication unit is not adjustable. Consequently, the entire communication unit may not extend over the shoe rim in boots and other high-rimmed shoes, and the communication unit may be disposed at a disadvantageously distance from the shoe rim in low shoes.

Another advantage of the foot sensor device **400** of the present invention is that wiring **120** is thin and is easily contained within the shoe. The wide spine of the device disclosed by Haugland, et al., is bulky, such that the user is subject to the distraction of sensing the spine within the shoe, even to the point of experiencing discomfort.

Perhaps most significantly, foot sensor device **400** may be advantageously fixed to the rim of the shoe in a semi-permanent fashion, i.e., even during those periods in which the user is not undergoing neuroprosthetic gait enhancement. Thus, prior to activating or reactivating the device, no additional donning procedure is required.

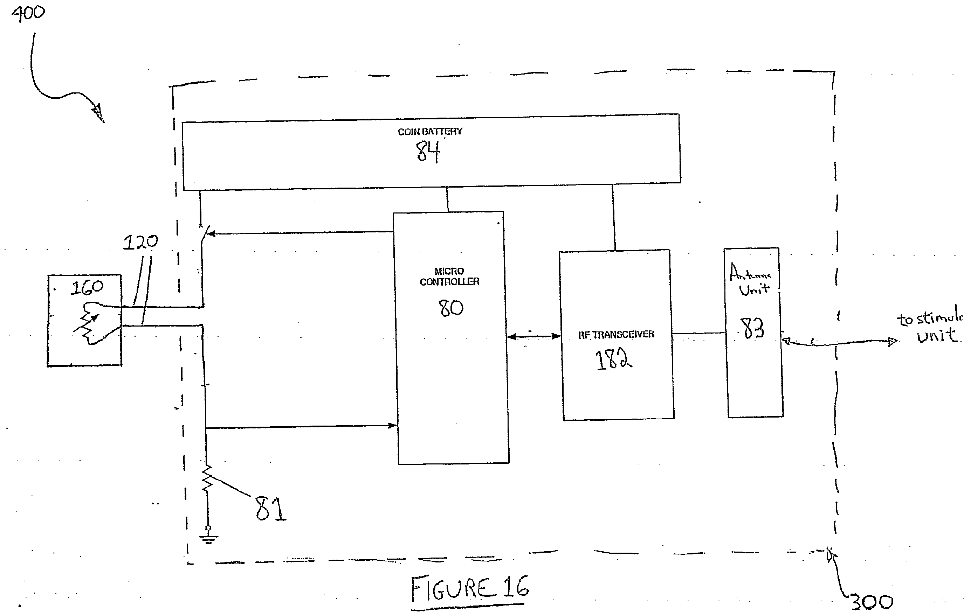

Figure 16 is a schematic electronic diagram of inventive foot sensor device **400.** Sensor element **160** is connected to, and preferably powered by, electronics or communication unit **300** by means of wiring **120.** Communication unit **300** includes a microcontroller **80,** a radio frequency (RF) transceiver **82,** and an antenna unit **83** having a matching network for converting the signal from the wired medium to a wireless medium, and from the wireless medium to the wired medium.

The resistance of sensor element **160** changes with the force applied thereon. In order to measure the actual resistance of sensor element **160,** foot sensor device **400** is equipped with a voltage divider consisting of sensor element **160** and a bias resistor **81** preferably disposed in unit **300.** When a voltage is applied to the voltage divider, the voltage is divided according to the resistance ratio between sensor element **160** and bias resistor **81.** This voltage is measured in order to assess the resistance of sensor element **160.**

Communication unit **300** is also equipped with a small coin battery **84** that provides power to microcontroller **80,** RF transceiver **82,** and sensor element **160.**

Microcontroller **80** controls and monitor the operation of foot sensor device **400** and executes the algorithms thereof. Preferably, microcontroller **80** communicates with RF transceiver **82** via a Serial Peripheral Interface (SPI).

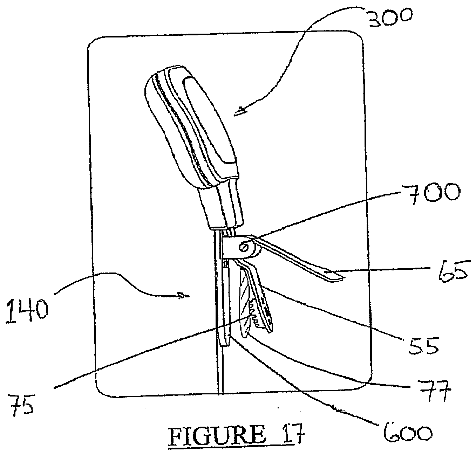

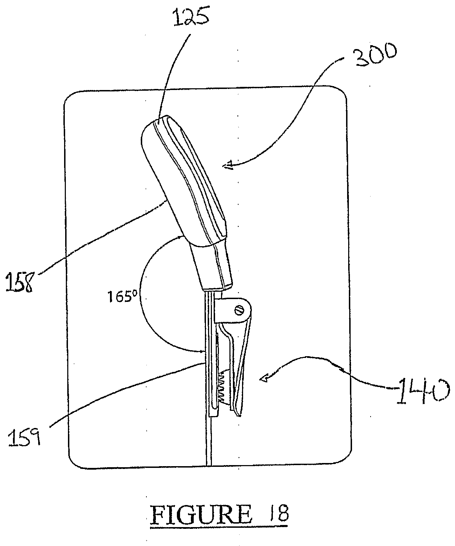

Referring now to Figures 17 and 18, clamp unit **140** is designed for facile and fully reversible attachment and disattachment of communication unit **300** to the rim of a shoe, such that even a hemiplegic patient can perform these functions using a single hand.

Clamp unit **140** includes an external (i.e., distal to the shoe) arm or jaw **55,** an internal arm or jaw **600** and a locking lever **65** having a fulcrum **700** for closing and locking together jaws **55** and **600.** Preferably, when clamp unit **140** is in an open position, jaws **55** and **600** are positioned in a pre-defined angle. The magnitude of this angle is important, since it determines the opening range of the clamp, to fit shoe rims of varying width.

Preferably, arm **55** includes a spring adapted such that clamp unit **140** is in a normally open condition.

Preferably, clamp unit **140** is further adapted such that when clamp unit **140** is in the closed position, with no article clamped between jaws **55** and **600,** a small gap (typically less than or equal to one millimeter) exists between jaws **55** and **600.** This eliminates the need to apply a strong force in opening clamp unit **140.**

Teeth **75,** disposed on at least one of jaws **55** and **600,** ensure a firm grip of clamp unit **140** on the shoe rim, such that clamp unit **140** is inherently adaptable to a wide range of rim thicknesses, contours, and textures. Locking lever **65** serves to fix jaws **55** and **600** to the rim of the shoe.

Internal jaw **600** has a thin profile, such that when disposed inside shoe **550** of the user, internal jaw **600** does not to protrude into the skin of the foot and cause discomfort to the user.

Preferably, a reversibly attached, thin, flexible spacer such as spacer 77 is disposed between jaws **55** and **600,** in order to protect the rim of the shoe from the local pressures exerted by teeth **75.**

Typically, clamp unit **140** is permanently and rigidly associated with communication unit **300.** The connection of clamp unit **140** to communication unit **300** is achieved by an element (not shown) that, from clamp unit **140,** extends into communication unit **300** and is secured therein.

It must be emphasized that other embodiments of clamps and fastening units will be apparent to those skilled in the art. For example, the clamp could have fixed arms that grip the shoe rim by a constant spring force, without an opening and closing mechanism.

As used herein in the specification and in the claims section that follows, the term "casing", with respect to the electronic communication unit, refers to the at least semi-rigid casing enveloping at least a portion of the electronic communication unit.

As used herein in the specification and in the claims section that follows, the term "housing" and the like, with respect to the electronic communication unit, is meant to include any rigid or semi-rigid projections of the housing, and is meant to include any elements that are at least semi-rigidly attached to the casing. The term "housing" is specifically meant to include rigid and semi-rigid spines attached to the electronic communication unit, such as the element disclosed in U.S. Design Patent D 494,273 to Haugland, et al.

As used herein in the specification and in the claims section that follows, the term "shoe" is meant to include boots, slippers, or sandals having an at least semi-rigid position for fastening the fastening unit. Typically, this at least semi-rigid position is the rim of the shoe. Preferably, the shoe has rigid or at least semi-rigid sole and a covering for firmly containing a foot therein.

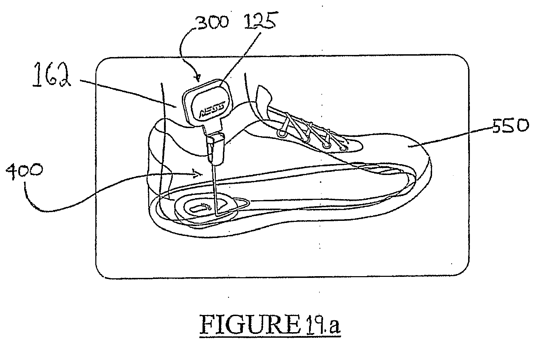

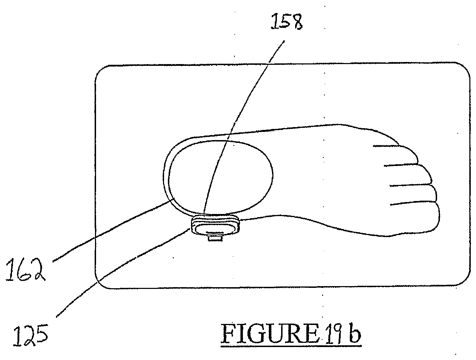

Figure 19a is a schematic, three-dimensional view of the inventive foot sensor device **400** mounted on shoe **550.** Figure 19b is a schematic, three-dimensional top view of Figure 19a. These figures collectively show an inner face **158** of housing **125** (of communication unit **300)** hugging an inner calf **162** of the user. The surface of the casing has a small, predetermined curvature that is designed to match and hug the natural curvature of the leg, thereby improving comfort and preventing chafing of communication unit **300** against the skin.

Flexibility, or a degree of freedom, with respect to the leg, is imparted to communication unit **300** by the flexibility of the shoe rim.

Alternatively, communication unit **300** may be connected to clamp **140** by means of an elastomeric hinge to allow extra flexibility of the casing of communication unit **300** against the foot. The electronic casing may also include a hinge that allows calibrating the angle of the electronic casing against the foot for personal adjustment with respect to specific users and specific shoe dimensions.

Referring back to Figure 18, inner face **158** of housing **125** and an inner face **159** of clamp unit **140** (internal arm **600**) form an angle of about 165°. After extensive tests were performed on users having widely varying physical characteristics, the inventors discovered that within a narrow range of angles, chafing and pressure of housing **125** against the leg of the user is substantially insignificant. In addition, within this narrow range of angles, housing **125** does not protrude away from the leg in an unreasonable fashion, such that housing **125** is protected from bumping into, or being caught by objects during gait. We have found this narrow range of angles to be between 150° and 175°. More preferably the range of angles is between 155° to 160° and 175°, and most preferably, the range is between 160° and 170°.

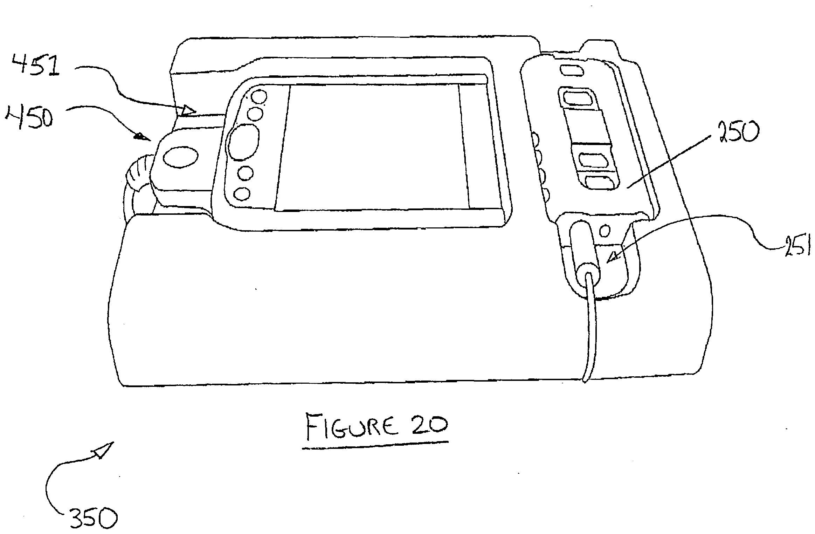

It is often desirable to configure the inventive FES orthosis system on-line, i.e., during usage of the orthosis by the patient. Figure 20 is a schematic perspective drawing of an inventive configuration cradle **350** for on-line configuration of the system by the clinician. Configuration cradle **350** has a mechanical and electrical receptacle **251** for receiving control unit **250,** and a mechanical and electrical receptacle **451** for receiving a personal digital assistant (PDA) **450.** Control unit **250** is described in greater detail hereinabove, with respect to Figure 11.

As is known in the art, PDAs such as PDA **450** are small, hand-held portable computers having a Central Processing Unit (CPU) and electronic memory, and are generally used for storing and organizing information and for providing tools for everyday tasks. The PDA currently being used in conjunction with this aspect of the present invention is operated by the "Windows Mobile 5" software of Microsoft®. PDA **450** preferably has a database containing a gait log and various personal parameters of the patient, and is programmed to configure the stimulation parameters of the electrical stimulation system.

Configuration cradle **350** enables PDA **450** and control unit **250** to be in digital (and preferably electrical) communication, such that the orthosis system can be configured on-line by the clinician during actual usage of the orthosis by the patient. In this arrangement, control unit **250** actually serves as the transmitter of PDA **450,** enabling PDA **450,** via control unit **250,** to communicate with and command the other components of the electrical stimulation system.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. An apparatus, comprising:
a semi-rigid frame (50) configured to substantially envelop a limb segment of a body, said frame (50) including a retaining element pair (12) including a flexible elongated retaining member (14) and an opposing flexible elongated member, the retaining element pair (12) configured to maintain a position of the frame (50) relative to the limb segment, the frame (50) including a housing (30) configured to be coupled to a stimulator unit (46);
a_soft inner layer (114) coupled to an inner surface of the frame (50);
a connector assembly (117, 117a, 123) coupled to the frame such that at least a portion of the connector assembly (117, 117a, 123) is disposed within an opening defined by at least one of the frame (50) or the soft inner layer (114); and
at least one surface electrode (94, 94A) configured to be supported by the frame (50) and configured to contact at least one stimulation point on a surface of the limb segment, said at least one surface electrode (94, 94A) configured to be electrically coupled to the stimulator unit (46) via the connector assembly (117, 117a, 123), the surface electrode (94, 94A) configured to provide electrical stimulation.

2. The apparatus of claim 1, wherein the flexible elongated member is from a plurality of flexible elongated members (10a-d), the retaining member (14) and the plurality of flexible elongated members (10a-d) are configured to conform to a contour of said surface in a substantially independent fashion, so as to maintain contact with said contour.

3. The apparatus of claim 2, wherein the plurality of flexible elongated members (10a-d) include at least three flexible elongated members extending circumferentially from the second side of the frame (50).

4. The apparatus of claim 1, further comprising:
a locking mechanism (34) configured to lock said at least one surface electrode (94, 94A) in contact with the stimulation point on said surface of the limb segment.

5. The apparatus of claim 1, wherein said retaining member (14) and the flexible elongated member (10c) are configured to independently move in response to changes in the surface of the limb segment such that a pressure exerted by said at least one surface electrode (94) against the stimulation point on said surface of the limb segment is substantially uniform.

6. The apparatus of claim 1, wherein the housing (30) is configured to receive said stimulator unit (46).

7. The apparatus of claim 6, wherein said housing (30) is configured to contain at least a portion of the stimulator unit (46) against the frame (50).

8. The apparatus of claim 1, further comprising:
said stimulator unit (46), wherein said stimulator unit (46) is configured to:
receive a signal from a sensor, the signal associated with a physical parameter relating to the limb segment, and
deliver, based on the signal received from said sensor, a stimulation signal to said surface electrode (94, 94A).

9. The apparatus of claim 8, wherein said stimulator unit (46) includes a radio frequency transceiver (228) configured to communicate with at least one of a stimulator control unit and said sensor.

10. The apparatus of claim 4, wherein said locking mechanism (34) includes at least one elastic strap configured to extend circumferentially around at least a portion of the frame (50) and between a first side of said frame (50) and a second side of said frame (50), the elastic strap configured to be reversibly fastened to a fastening element associated with said frame (50).

11. The apparatus of claim 1, wherein an upper portion of the frame (50) includes a locating surface (22) configured to conform to an inferior border of a patella of the body.

12. The apparatus of claim 1, wherein an upper portion of said frame (50) includes a locating surface (22) having a three-dimensional cup shape configured to matingly abut an inferior border of a patella of the body when the frame (50) is disposed about the limb segment.

13. The apparatus of claim 1, wherein the frame (50) is configured to be disposed about the limb segment with a single hand.

14. The apparatus of claim 10, wherein said fastening element includes a protuberance associated with said frame (50), the protuberance including the housing (30).

15. The apparatus of claim 14, wherein said housing (30) is configured to receive said stimulator unit (46).

16. The apparatus of claim 10, wherein an end portion of said at least one elastic strap includes a looped handle (54), said looped handle (54) configured to be reversibly fastened to said fastening element.

17. The apparatus of claim 1, wherein said housing (30) is disposed on a flexible portion of said frame (50), said flexible portion of the frame (50) configured to move independently of the retaining element pair (12).

18. The apparatus of claim 1, wherein said frame (50) includes at least one spring-loaded strip, said strip being radially spring-loaded towards the center of said frame (50).

19. The apparatus of claim 2, wherein at least one of said retaining member (14) or a flexible elongated member from the plurality of flexible elongated members (10a-d) has a width of between about 2 cm and about 4.5 cm.

20. The apparatus of claim 2, wherein at least one of said retaining member (14) or a flexible elongated member from the plurality of flexible elongated members (10a-d) has a width within a range of 8 percent of a height of the frame (50) and 60 percent of the height of the frame (50).

21. The apparatus of claim 1, wherein said at least one surface electrode (94, 94A) has a surface area (95) configured to contact said surface of the limb segment, said surface area being at least 9 cm².

22. The apparatus of claim 21, wherein said surface area (95) is at least 12 cm².

23. The apparatus of claim 14, wherein an end portion of said at least one elastic strap includes a looped handle (54), said looped handle (54) configured to removably engage a portion of the protuberance of said fastening element, said stimulator unit (46), said housing (30), and said looped handle (54) collectively forming a profile of a leg of the body when the looped handle (54) is fastened to the fastening element (34).

24. The apparatus of claim 1, wherein said stimulator unit (46) is a neuroprosthetic stimulator unit.

25. The apparatus of claim 1, further comprising an electrode assembly (90) which comprises the surface electrode (94, 94A) an electrode base (98, 98A), the electrode base configured to be coupled to the soft inner layer, the electrode base configured to couple the surface electrode to the soft inner layer, the frame and the soft inner layer collectively configured such that the electrode base is disposed at a predetermined position relative to the portion of the limb segment when the frame and the soft inner layer are disposed about the portion of the limb segment.

26. The apparatus of claim 25, wherein the electrode assembly includes a complementary conductive connector (122) and a conductive wire coupled between the electrode base (98, 98A), and the complementary conductive connector (122), wherein the at least one surface electrode (94, 94A) is configured to be electrically coupled to the stimulator unit (46) via the connector assembly (117, 117a, 123) and the complementary conductive connector (122).

27. The apparatus of claim 25, wherein the connector assembly (117, 117a, 123) includes an attachment mechanism for fixing a position of said electrode assembly (90) with respect to said frame (50) comprising a first mechanical fitting (117a) associated with the inner face of said frame (50) and a complementary second mechanical fitting (117) associated with the electrode assembly (90) for reversibly attaching said electrode assembly (90) to, and reversibly detaching said electrode assembly (90) from, said first mechanical fitting (117a),
wherein said first and second mechanical fittings (117, 117a) are a first connector and second connector selected from the group consisting of snaps and hook and loop fasteners.

28. The apparatus of claim 25, wherein:
the surface electrode (94, 94A) includes a first surface (95); and
the electrode base (98, 98A) has a surface (99) configured to receive and engage a second surface (97) of said surface electrode (94, 94A), said second surface (97) being opposite said first surface (95).

29. The apparatus of claim 25, wherein said electrode base (98, 98A) has a rim (101) defining an opening configured to receive said surface electrode (94, 94A) and maintain a position of the surface electrode (94, 94A) relative to the electrode base (98, 98A).

30. The apparatus of claim 27, wherein said second surface (97) of said surface electrode (94, 94A) includes a hydrogel-containing surface, and said surface (99) of said electrode base (98, 98A) includes at least one patch of hook fasteners (103) configured to couple said hydrogel-containing surface to said electrode base (98, 98A).

31. The apparatus of claim 1, wherein said soft inner layer (114, 118) is configured to be reversibly attached to the frame (50).

32. The apparatus of claim 27, further comprising:
a cover (107) attached to said electrode base (98, 98A), a perimeter (127) of said surface electrode (94, 94A) configured to be disposed between the cover (107) and a portion of the electrode base (98, 98A).

33. The apparatus of claim 32, wherein said cover (107) defines an opening such that a central portion said first surface (95) of the surface electrode (94, 94A) is exposed when the perimeter (127) of the surface electrode (94, 94A) is disposed between the cover (107) and the portion of the electrode base (98, 98A).

34. The apparatus of claim 32, wherein said perimeter (127) includes hydrogel.

35. The apparatus of claim 1, wherein the connector assembly (117, 117A, 123) includes a first connector (117A) associated with at least one of said inner surface of said frame (50) and the soft inner layer (114), and a second connector (117) complementary to said first connector (117A), the first connector (117A) and the second connector (117) configured to reversibly attach said at least one surface electrode (94, 94A) to at least one of the frame (50) and the soft inner layer (114).

36. The apparatus of claim 34, further comprising:
a detachable layer (118) configured to cover at least a portion of said soft inner layer (114), said detachable layer (118) having a third connector (124, 134) complementary to a fourth connector (135), the fourth connector (135) associated with at least one of said frame and said soft inner layer (114), the third connector (124, 134) and the fourth connector (135) configured to reversibly couple said detachable layer (118) to at least one of said frame (50) and said soft inner layer (114).

## Patentansprüche

1. Vorrichtung, die Folgendes umfasst:
einen halbstarren Rahmen (50), der zur wesentlichen Umhüllung eines Gliedmaßensegments eines Körpers konfiguriert ist, wobei der Rahmen (50) ein Halteelementpaar (12) einschließt, das ein flexibles längliches Halteteil (14) und ein gegenüberliegendes flexibles längliches Teil einschließt, das Halteelementpaar (12) zur Aufrechterhaltung einer Position des Rahmens (50) relativ zum Gliedmaßensegment konfiguriert ist, der Rahmen (50) ein Gehäuse (30) einschließt, das zur Verbindung mit einer Stimulatoreinheit (46) konfiguriert ist;
eine weiche innere Schicht (114), die mit einer inneren Oberfläche des Rahmens (50) verbunden ist;
eine Verbindungsanordnung (117, 117a, 123), die mit dem Rahmen derart verbunden ist, dass zumindest ein Teil der Verbindungsanordnung (117, 117a, 123) innerhalb einer Öffnung angeordnet ist, die durch mindestens eines von dem Rahmen (50) oder der weichen inneren Schicht (114) definiert ist; und
mindestens eine Oberflächenelektrode (94, 94A), die zur Unterstützung durch den Rahmen (50) konfiguriert ist und zum Kontakt von mindestens einem Stimulationspunkt auf einer Oberfläche des Gliedmaßensegments konfiguriert ist, wobei die mindestens eine Oberflächenelektrode (94, 94A) zur elektrischen Verbindung mit der Stimulatoreinheit (46) über die Verbindungsanordnung (117, 117a, 123) konfiguriert ist, die Oberflächenelektrode (94, 94A) zur Bereitstellung von elektrischer Stimulation konfiguriert ist.

2. Vorrichtung nach Anspruch 1, wobei das flexible längliche Teil von einer Vielzahl von flexiblen länglichen Teilen (10a-d) kommt, das Halteteil (14) und die Vielzahl von flexiblen länglichen Teilen (10a-d) zur Übereinstimmung mit einer Kontur der Oberfläche in einer im Wesentlichen unabhängigen Weise konfiguriert sind, um den Kontakt mit der Kontur aufrechtzuerhalten.

3. Vorrichtung nach Anspruch 2, wobei die Vielzahl von flexiblen länglichen Teilen (10a-d) mindestens drei flexible längliche Teile einschließt, die sich umlaufend von der zweiten Seite des Rahmens (50) erstrecken.

4. Vorrichtung nach Anspruch 1, die weiter Folgendes umfasst:
einen Sperrmechanismus (34), der zur Sperrung der mindestens einen Oberflächenelektrode (94, 94A) im Kontakt mit dem Stimulationspunkt auf der Oberfläche des Gliedmaßensegments konfiguriert ist.

5. Vorrichtung nach Anspruch 1, wobei das Halteteil (14) und das flexible längliche Teil (10c) zur unabhängigen Bewegung als Reaktion auf Veränderungen der Oberfläche des Gliedmaßensegments konfiguriert sind, sodass ein Druck, der von der mindestens einen Oberflächenelektrode (94) gegen den Stimulationspunkt auf der Oberfläche des Gliedmaßensegments ausgeübt wird, im Wesentlichen gleichmäßig ist.

6. Vorrichtung nach Anspruch 1, wobei das Gehäuse (30) zur Aufnahme der Stimulatoreinheit (46) konfiguriert ist.

7. Vorrichtung nach Anspruch 6, wobei das Gehäuse (30) zur Einschließung von mindestens einem Teil der Stimulatoreinheit (46) gegen den Rahmen (50) konfiguriert ist.

8. Vorrichtung nach Anspruch 1, die weiter Folgendes umfasst:
die Stimulatoreinheit (46), wobei die Stimulatoreinheit (46) für Folgendes konfiguriert ist:
Empfang eines Signals von einem Sensor, wobei das Signal mit einem physischen Parameter, der mit dem Gliedmaßensegment in Beziehung steht, verbunden ist, und
Abgabe, basierend auf dem von dem Sensor empfangenen Signal, eines Stimulationssignals an die Oberflächenelektrode (94, 94A).

9. Vorrichtung nach Anspruch 8, wobei die Stimulatoreinheit (46) einen Hochfrequenzempfänger (228) einschließt, der zur Kommunikation mit mindestens einem von einer Stimulatorsteuereinheit und dem Sensor konfiguriert ist.

10. Vorrichtung nach Anspruch 4, wobei der Sperrmechanismus (34) mindestens ein elastisches Band einschließt, das konfiguriert ist, um sich umlaufend um mindestens ein Teil des Rahmens (50) und zwischen einer ersten Seite des Rahmens (50) und einer zweiten Seite des Rahmens (50) zu erstrecken, wobei das elastische Band zur reversiblen Befestigung an ein Befestigungselement, das mit dem Rahmen (50) verbunden ist, konfiguriert ist.

11. Vorrichtung nach Anspruch 1, wobei ein oberes Teil des Rahmens (50) eine Anlagefläche (22) einschließt, die zur Übereinstimmung mit einem unteren Rand einer Kniescheibe des Körpers konfiguriert ist.

12. Vorrichtung nach Anspruch 1, wobei ein oberes Teil des Rahmens (50) eine Anlagefläche (22) einschließt, die eine dreidimensionale Becherform aufweist, die zum verbindenden Aneinanderstoßen eines unteren Rands einer Kniescheibe des Körpers konfiguriert ist, wenn der Rahmen (50) um das Gliedmaßensegment angeordnet ist.

13. Vorrichtung nach Anspruch 1, wobei der Rahmen (50) zur Anordnung um das Gliedmaßensegment mit einer einzigen Hand konfiguriert ist.

14. Vorrichtung nach Anspruch 10, wobei das Befestigungselement einen Vorsprung einschließt, der mit dem Rahmen (50) verbunden ist, der Vorsprung das Gehäuse (30) einschließt.

15. Vorrichtung nach Anspruch 14, wobei das Gehäuse (30) zur Aufnahme der Stimulatoreinheit (46) konfiguriert ist.

16. Vorrichtung nach Anspruch 10, wobei ein Endteil des mindestens einen elastischen Bands einen gewundenen Griff (54) einschließt, wobei der gewundene Griff (54) zur reversiblen Befestigung an das Befestigungselement konfiguriert ist.

17. Vorrichtung nach Anspruch 1, wobei das Gehäuse (30) an einem flexiblen Teil des Rahmens (50) angeordnet ist, wobei das flexible Teil des Rahmens (50) zur unabhängigen Bewegung des Halteelementpaars (12) konfiguriert ist.

18. Vorrichtung nach Anspruch 1, wobei der Rahmen (50) mindestens einen federgespannten Streifen einschließt, wobei der Streifen radial in Richtung der Mitte des Rahmens (50) federgespannt ist.

19. Vorrichtung nach Anspruch 2, wobei mindestens eines von dem Halteteil (14) oder einem flexiblen länglichen Teil aus der Vielzahl von flexiblen länglichen Teilen (10a-d) eine Breite zwischen etwa 2 cm und etwa 4,5 cm aufweist.

20. Vorrichtung nach Anspruch 2, wobei mindestens eines von dem Halteteil (14) oder einem flexiblen länglichen Teil aus der Vielzahl von flexiblen länglichen Teilen (10a-d) eine Breite in einem Bereich von 8 Prozent einer Höhe des Rahmens (50) und 60 Prozent der Höhe des Rahmens (50) aufweist.

21. Vorrichtung nach Anspruch 1, wobei die mindestens eine Oberflächenelektrode (94, 94A) eine Oberflächenfläche (95) aufweist, die zum Kontakt der Oberfläche des Gliedmaßensegments konfiguriert ist, wobei die Oberflächenfläche mindestens 9 cm² beträgt.

22. Vorrichtung nach Anspruch 21, wobei die Oberflächenfläche (95) mindestens 12 cm² beträgt.

23. Vorrichtung nach Anspruch 14, wobei ein Endteil des mindestens einen elastischen Bands einen gewundenen Griff (54) einschließt, wobei der gewundene Griff (54) konfiguriert ist, um in ein Teil des Vorsprungs des Befestigungselements entfernbar einzugreifen, die Stimulatoreinheit (46), das Gehäuse (30) und der gewundene Griff (54) zusammen ein Profil eines Beins des Körpers bilden, wenn der gewundene Griff (54) an dem Befestigungselement (34) befestigt ist.

24. Vorrichtung nach Anspruch 1, wobei die Stimulatoreinheit (46) eine neuroprothetische Stimulatoreinheit ist.

25. Vorrichtung nach Anspruch 1, die weiter eine Elektrodenanordnung (90) umfasst, die die Oberflächenelektrode (94, 94A), ein Elektrodengrundteil (98, 98A) umfasst, wobei das Elektrodengrundteil zur Verbindung mit der weichen inneren Schicht konfiguriert ist, das Elektrodengrundteil zur Verbindung der Oberflächenelektrode mit der weichen inneren Schicht konfiguriert ist, der Rahmen und die weiche innere Schicht zusammen derart konfiguriert sind, dass das Elektrodengrundteil an einer vorbestimmten Position relativ zum Teil des Gliedmaßensegments angeordnet ist, wenn der Rahmen und die weiche innere Schicht um das Teil des Gliedmaßensegments angeordnet sind.

26. Vorrichtung nach Anspruch 25, wobei die Elektrodenanordnung einen komplementären leitfähigen Verbinder (122) und einen leitfähigen Draht, der zwischen dem Elektrodengrundteil (98, 98A) und dem komplementären leitfähigen Verbinder (122) verbunden ist, einschließt, wobei die mindestens eine Oberflächenelektrode (94, 94A) zur elektrischen Verbindung mit der Stimulatoreinheit (46) über die Verbindungsanordnung (117, 117a, 123) und den komplementären leitfähigen Verbinder (122) konfiguriert ist.

27. Vorrichtung nach Anspruch 25, wobei die Verbindungsanordnung (117, 117a, 123) einen Anbringungsmechanismus für die Fixierung einer Position der Elektrodenanordnung (90) in Bezug auf den Rahmen (50) einschließt, der eine erste mechanische Befestigung (117a), die mit der inneren Seite des Rahmens (50) verbunden ist, und eine komplementäre zweite mechanische Befestigung (117), die mit der Elektrodenanordnung (90) für die reversible Anbringung der Elektrodenanordnung (90) an die und reversible Abtrennung der Elektrodenanordnung (90) von der ersten mechanischen Befestigung (117a) verbunden ist, umfasst,
wobei die erste und zweite mechanische Befestigung (117, 117a) ein erster Verbinder und zweiter Verbinder sind, die aus der Gruppe ausgewählt sind, die aus Schnappverschlüssen und Haken- und Schlaufenverschlüssen besteht.

28. Vorrichtung nach Anspruch 25, wobei:
die Oberflächenelektrode (94, 94A) eine erste Oberfläche (95) einschließt; und
das Elektrodengrundteil (98, 98A) eine Oberfläche (99) aufweist, die zur Aufnahme und zum Eingriff in eine zweite Oberfläche (97) der Oberflächenelektrode (94, 94A) konfiguriert ist, wobei die zweite Oberfläche (97) der ersten Oberfläche (95) gegenüberliegt.

29. Vorrichtung nach Anspruch 25, wobei das Elektrodengrundteil (98, 98A) einen Rand (101) aufweist, der eine Öffnung definiert, die zur Aufnahme der Oberflächenelektrode (94, 94A) und zur Aufrechterhaltung einer Position der Oberflächenelektrode (94, 94A) relativ zum Elektrodengrundteil (98, 98A) konfiguriert ist.

30. Vorrichtung nach Anspruch 27, wobei die zweite Oberfläche (97) der Oberflächenelektrode (94, 94A) eine Hydrogel enthaltende Oberfläche einschließt und die Oberfläche (99) des Elektrodengrundteils (98, 98A) mindestens eine Stelle von Hakenverschlüssen (103) einschließt, die zur Verbindung der Hydrogel enthaltenden Oberfläche mit dem Elektrodengrundteil (98, 98A) konfiguriert sind.

31. Vorrichtung nach Anspruch 1, wobei die weiche innere Schicht (114, 118) zur reversiblen Anbringung an den Rahmen (50) konfiguriert ist.

32. Vorrichtung nach Anspruch 27, die weiter Folgendes umfasst:
eine Abdeckung (107), die an das Elektrodengrundteil (98, 98A) angebracht ist, eine Umgrenzung (127) der Oberflächenelektrode (94, 94A), die zur Anordnung zwischen der Abdeckung (107) und einem Teil des Elektrodengrundteils (98, 98A) konfiguriert ist.

33. Vorrichtung nach Anspruch 32, wobei die Abdeckung (107) eine Öffnung definiert, sodass ein mittlerer Teil der ersten Oberfläche (95) der Oberflächenelektrode (94, 94A) exponiert ist, wenn die Umgrenzung (127) der Oberflächenelektrode (94, 94A) zwischen der Abdeckung (107) und dem Teil des Elektrodengrundteils (98, 98A) angeordnet ist.

34. Vorrichtung nach Anspruch 32, wobei die Umgrenzung (127) Hydrogel einschließt.

35. Vorrichtung nach Anspruch 1, wobei die Verbindungsanordnung (117, 117A, 123) Folgendes einschließt: einen ersten Verbinder (117A), der mit mindestens einer von der inneren Oberfläche des Rahmens (50) und der weichen inneren Schicht (114) verbunden ist, und einen zweiten Verbinder (117), der zum ersten Verbinder (117A) komplementär ist, wobei der erste Verbinder (117A) und der zweite Verbinder (117) zur reversiblen Anbringung der mindestens einen Oberflächenelektrode (94, 94A) an mindestens eines von dem Rahmen (50) und der weichen inneren Schicht (114) konfiguriert sind.

36. Vorrichtung nach Anspruch 34, die weiter Folgendes umfasst:
eine abnehmbare Schicht (118), die zur Abdeckung von mindestens einem Teil der weichen inneren Schicht (114) konfiguriert ist, wobei die abnehmbare Schicht (118) einen dritten Verbinder (124, 134) aufweist, der zu einem vierten Verbinder (135) komplementär ist, wobei der vierte Verbinder (135) mit mindestens einem von dem Rahmen und der weichen inneren Schicht (114) verbunden ist, der dritte Verbinder (124, 134) und der vierte Verbinder (135) zur reversiblen Verbindung der abnehmbaren Schicht (118) mit mindestens einem von dem Rahmen (50) und der weichen inneren Schicht (114) konfiguriert sind.

## Revendications

1. Appareil, comportant :
un châssis semi-rigide (50) configuré pour envelopper sensiblement un segment de membre d'un corps, ledit châssis (50) comprenant une paire d'éléments de retenue (12) comprenant un organe de retenue allongé flexible (14) et un organe allongé flexible opposé, la paire d'éléments de retenue (12) étant configurée pour maintenir une position du châssis (50) par rapport au segment de membre, le châssis (50) comprenant un boîtier (30) configuré pour être accouplé à une unité de stimulateur (46) ;
une couche intérieure souple (114) accouplée à une surface intérieure du châssis (50) ;
un ensemble connecteur (117, 117a, 123) accouplé au châssis de telle sorte qu'au moins une partie de l'ensemble connecteur (117, 117a, 123) soit disposée à l'intérieur d'une ouverture délimitée par au moins l'un parmi le châssis (50) ou la couche intérieure souple (114) ; et
au moins une électrode de surface (94, 94A) configurée pour être supportée par le châssis (50) et configurée pour venir en contact avec au moins un point de stimulation sur une surface du segment de membre, ladite au moins une électrode de surface (94, 94A) étant configurée pour être accouplée de manière électrique à l'unité de stimulateur (46) par l'intermédiaire de l'ensemble connecteur (117, 117a, 123), l'électrode de surface (94, 94A) étant configurée pour fournir une stimulation électrique.

2. Appareil selon la revendication 1, dans lequel l'organe allongé flexible est constitué d'une pluralité d'organes allongés flexibles (10a à d), l'organe de retenue (14) et la pluralité d'organes allongés flexibles (10a à d) étant configurés pour se conformer à un contour de ladite surface d'une manière sensiblement indépendante, de façon à maintenir un contact avec ledit contour.

3. Appareil selon la revendication 2, dans lequel la pluralité d'organes allongés flexibles (10 à a d) comprend au moins trois organes allongés flexibles qui s'étendent circonférentiellement depuis le deuxième côté du châssis (50).

4. Appareil selon la revendication 1, comportant en outre :
un mécanisme de verrouillage (34) configuré pour verrouiller ladite au moins une électrode de surface (94, 94A) en contact avec le point de stimulation sur ladite surface du segment de membre.

5. Appareil selon la revendication 1, dans lequel ledit organe de retenue (14) et l'organe allongé flexible (10c) sont configurés pour se déplacer indépendamment en réponse à des changements de la surface du segment de membre de telle sorte qu'une pression exercée par ladite au moins une électrode de surface (94) contre le point de stimulation sur ladite surface du segment de membre soit sensiblement uniforme.

6. Appareil selon la revendication 1, dans lequel le boîtier (30) est configuré pour recevoir ladite unité de stimulateur (46).

7. Appareil selon la revendication 6, dans lequel ledit boîtier (30) est configuré pour contenir au moins une partie de l'unité de stimulateur (46) contre le châssis (50).

8. Appareil selon la revendication 1, comportant en outre :
ladite unité de stimulateur (46), ladite unité de stimulateur (46) étant configurée pour :
recevoir un signal d'un capteur, le signal étant associé à un paramètre physique se rapportant au segment de membre, et
délivrer, sur la base du signal reçu depuis ledit capteur, un signal de stimulation à ladite électrode de surface (94, 94A).

9. Appareil selon la revendication 8, dans lequel ladite unité de stimulateur (46) comprend un émetteur-récepteur de radiofréquence (228) configuré pour communiquer avec au moins l'un parmi une unité de commande de stimulateur et ledit capteur.

10. Appareil selon la revendication 4, dans lequel ledit mécanisme de verrouillage (34) comprend au moins une sangle élastique configurée pour s'étendre circonférentiellement autour d'au moins une partie du châssis (50) et entre un premier côté dudit châssis (50) et un deuxième côté dudit châssis (50), la sangle élastique étant configurée pour être fixée de manière réversible à un élément de fixation associé audit châssis (50).

11. Appareil selon la revendication 1, dans lequel une partie supérieure du châssis (50) comprend une surface de positionnement (22) configurée pour se conformer à un bord inférieur d'une rotule du corps.

12. Appareil selon la revendication 1, dans lequel une partie supérieure dudit châssis (50) comprend une surface de positionnement (22) en forme de cupule tridimensionnelle configurée pour venir buter en correspondance avec un bord inférieur d'une rotule du corps lorsque le châssis (50) est disposé autour du segment de membre.

13. Appareil selon la revendication 1, dans lequel le châssis (50) est configuré pour être disposé autour du segment de membre avec une seule main.

14. Appareil selon la revendication 10, dans lequel ledit élément de fixation comprend une protubérance associée audit châssis (50), la protubérance comprenant le boîtier (30).

15. Appareil selon la revendication 14, dans lequel ledit boîtier (30) est configuré pour recevoir ladite unité de stimulateur (46).

16. Appareil selon la revendication 10, dans lequel une partie d'extrémité de ladite au moins une sangle élastique comprend une poignée en boucle (54), ladite poignée en boucle (54) étant configurée pour être fixée de manière réversible audit élément de fixation.

17. Appareil selon la revendication 1, dans lequel ledit boîtier (30) est disposé sur une partie flexible dudit châssis (50), ladite partie flexible du châssis (50) étant configurée pour se déplacer indépendamment de la paire d'éléments de retenue (12).

18. Appareil selon la revendication 1, dans lequel ledit châssis (50) comprend au moins une bande sollicitée par ressort, ladite bande étant sollicitée par ressort radialement contre le centre dudit châssis (50).

19. Appareil selon la revendication 2, dans lequel au moins l'un parmi ledit organe de retenue (14) ou un organe allongé flexible de la pluralité d'organes allongés flexibles (10a à d) a une largeur comprise entre environ 2 cm et environ 4,5 cm.

20. Appareil selon la revendication 2, dans lequel au moins l'un parmi ledit organe de retenue (14) ou un organe allongé flexible de la pluralité d'organes allongés flexibles (10a à d) a une largeur dans une plage comprise entre 8 pour cent d'une hauteur du châssis (50) et 60 pour cent de la hauteur du châssis (50).

21. Appareil selon la revendication 1, dans lequel ladite au moins une électrode de surface (94, 94A) présente une zone de surface (95) configurée pour venir en contact avec ladite surface du segment de membre, ladite zone de surface étant d'au moins 9 cm².

22. Appareil selon la revendication 21, dans lequel ladite zone de surface (95) est d'au moins 12 cm².

23. Appareil selon la revendication 14, dans lequel une partie d'extrémité de ladite au moins une bande élastique comprend une poignée en boucle (54), ladite poignée en boucle (54) étant configurée pour se mettre en prise de manière amovible avec une partie de la protubérance dudit élément de fixation, ladite unité de stimulateur (46), ledit boîtier (30), et ladite poignée en boucle (54) formant collectivement un profil d'une jambe du corps lorsque la poignée en boucle (54) est fixée à l'élément de fixation (34).

24. Appareil selon la revendication 1, dans lequel ladite unité de stimulateur (46) est une unité de stimulateur neuroprothétique.

25. Appareil selon la revendication 1, comportant en outre un ensemble électrode (90) qui comporte l'électrode de surface (94, 94A), une base d'électrode (98, 98A), la base d'électrode étant configurée pour être accouplée à la couche intérieure souple, la base d'électrode étant configurée pour accoupler l'électrode de surface à la couche intérieure souple, le châssis et la couche intérieure souple étant collectivement configurés de telle sorte que la base d'électrode soit disposée à une position prédéterminée par rapport à la partie du segment de membre lorsque le châssis et la couche intérieure souple sont disposés autour de la partie du segment de membre.

26. Appareil selon la revendication 25, dans lequel l'ensemble électrode comprend un connecteur conducteur complémentaire (122) et un fil métallique conducteur accouplé entre la base d'électrode (98, 98A), et le connecteur conducteur complémentaire (122), dans lequel ladite au moins une électrode de surface (94, 94A) est configurée pour être électriquement accouplée à l'unité de stimulateur (46) par l'intermédiaire de l'ensemble connecteur (117, 117a, 123) et du connecteur conducteur complémentaire (122).

27. Appareil selon la revendication 25, dans lequel l'ensemble connecteur (117, 117a, 123) comprend un mécanisme d'attache permettant de fixer une position dudit ensemble électrode (90) par rapport audit châssis (50) comportant une première garniture mécanique (117a) associée à la face intérieure dudit châssis (50) et une deuxième garniture mécanique complémentaire (117) associée à l'ensemble électrode (90) permettant d'attacher de manière réversible ledit ensemble électrode (90) à ladite première garniture mécanique (117a) et de détacher de manière réversible ledit ensemble électrode (90) de ladite première garniture mécanique (117a),
dans lequel lesdites première et deuxième garnitures mécaniques (117, 117a) sont un premier connecteur et un deuxième connecteur sélectionnés dans le groupe constitué par des encliquetages et des fixations à crochets et à boucles.

28. Appareil selon la revendication 25, dans lequel:
l'électrode de surface (94, 94A) comporte une première surface (95) ; et
la base d'électrode (98, 98A) présente une surface (99) configurée pour recevoir et se mettre en prise avec une deuxième surface (97) de ladite électrode de surface (94, 94A), ladite deuxième surface (97) étant opposée à ladite première surface (95).

29. Appareil selon la revendication 25, dans lequel ladite base d'électrode (98, 98A) présente un rebord (101) délimitant une ouverture configurée pour recevoir ladite électrode de surface (94, 94A) et pour maintenir une position de l'électrode de surface (94, 94A) par rapport à la base d'électrode (98, 98A).

30. Appareil selon la revendication 27, dans lequel ladite deuxième surface (97) de ladite électrode de surface (94, 94A) comprend une surface contenant de l'hydrogel, et ladite surface (99) de ladite base d'électrode (98, 98A) comprend au moins une pièce de fixations à crochets (103) configurée pour accoupler ladite surface contenant de l'hydrogel à ladite base d'électrode (98, 98A).

31. Appareil selon la revendication 1, dans lequel ladite couche intérieure souple (114, 118) est configurée pour être attachée de manière réversible au châssis (50).

32. Appareil selon la revendication 27, comportant en outre :
un couvercle (107) attaché à ladite base d'électrode (98, 98A), un périmètre (127) de ladite électrode de surface (94, 94A) étant configuré pour être disposé entre le couvercle (107) et une partie de la base d'électrode (98, 98A).

33. Appareil selon la revendication 32, dans lequel ledit couvercle (107) délimite une ouverture telle qu'une partie centrale de ladite première surface (95) de l'électrode de surface (94, 94A) est exposée lorsque le périmètre (127) de l'électrode de surface (94, 94A) est disposé entre le couvercle (107) et la partie de la base d'électrode (98, 98A).

34. Appareil selon la revendication 32, dans lequel ledit périmètre (127) comprend de l'hydrogel.

35. Appareil selon la revendication 1, dans lequel l'ensemble connecteur (117, 117A, 123) comprend un premier connecteur (117A) associé à au moins l'une parmi la surface intérieure dudit châssis (50) et la couche intérieure souple (114), et un deuxième connecteur (117) complémentaire dudit premier connecteur (117A), le premier connecteur (117A) et le deuxième connecteur (117) étant configurés pour attacher de manière réversible ladite au moins une électrode de surface (94, 94A) à au moins l'un parmi le châssis (50) et la couche intérieure souple (114).

36. Appareil selon la revendication 34, comportant en outre :
une couche détachable (118) configurée pour recouvrir au moins une partie de ladite couche intérieure souple (114), ladite couche détachable (118) présentant un troisième connecteur (124, 134) complémentaire d'un quatrième connecteur (135), le quatrième connecteur (135) étant associé à au moins l'un parmi ledit châssis et ladite couche intérieure souple (114), le troisième connecteur (124, 134) et le quatrième connecteur (135) étant configurés pour accoupler de manière réversible ladite couche détachable (118) à au moins l'un parmi ledit châssis (50) et ladite couche intérieure souple (114).
